# EUROPEAN PATENT SPECIFICATION

(11) **EP 3 407 887 B1**
(45) Date of publication and mention of the grant of the patent: **01.09.2021**
(21) Application number: 17744934.5
(22) Date of filing: 27.01.2017
(51) Int. Cl.: A61K 31/454, A61K 31/706, A61K 31/496, A61P 35/02

(54) **CLINICAL REGIMEN FOR TREATING MYELODYSPLASTIC SYNDROME WITH PHOSPHATASE INHIBITOR**
KLINISCHES REGIME ZUR BEHANDLUNG DES MYELODYSPLASTISCHEN SYNDROMS MIT PHOSPHATASEINHIBITOR
RÉGIME CLINIQUE POUR LE TRAITEMENT DU SYNDROME MYÉLODYSPLASIQUE AVEC UN INHIBITEUR DE PHOSPHATASE

(30) Priority: 27.01.2016 US 201662287858 P
(43) Date of publication of application: 05.12.2018
(73) Proprietor: Lixte Biotechnology, Inc., East Setauket, NY 11733 (US); H. Lee Moffitt Cancer Center And Research Institute, Inc., Tampa, FL 33612 (US)
(72) Inventor: LIST, Alan F., Tampa, FL 33647 (US); SALLMAN, David A., Tampa, FL 33647 (US); KOVACH, John S., East Setauket, NY 11733 (US)
(74) Representative: Tostmann, Holger Carl
(86) International application number: PCT/US2017/015237
(87) International publication number: WO 2017/132445

(56) References cited:
- WO-A1-2016/014783
- US-A1- 2015 045 373
- US-A1- 2015 224 083
- US-A1- 2016 074 390
- CHAO HU ET AL: "PP2A inhibition from LB100 therapy enhances daunorubicin cytotoxicity in secondary acute myeloid leukemia via miR-181b-1 upregulation", SCIENTIFIC REPORTS, vol. 7, no. 1, 6 June 2017 (2017-06-06), XP055612703, DOI: 10.1038/s41598-017-03058-4
- BAI ET AL.: 'Inhibition of Protein Phosphatase 2A Enhances Cytotoxicity and Accessibility of Chemotherapeutic Drugs to Hepatocellular Carcinomas' MOLECULAR CANCER THERAPEUTICS vol. 13, no. 8, 17 May 2014, pages 2062 - 2072, XP055401986
- HONG ET AL.: 'LB100, a small molecule inhibitor of PP2A with potent chemo- and radio-sensitizing potential' CANCER BIOLOGY & THERAPY vol. 16, no. 6, 21 April 2015, pages 821 - 833, XP055401956

## Description

### Background of the Invention

Myelodysplastic syndromes (MDS) are hematopoietic stem cell malignancies with a rising prevalence owing to the aging of the American population. MDS comprise a group of malignant hematologic disorders associated with impaired erythropoiesis, dysregulated myeloid differentiation and increased risk for acute myeloid leukemia (AML) transformation. The incidence of MDS is increasing with 15,000 to 20,000 new cases each year in the United States and large numbers of patients requiring chronic blood transfusions. Ineffective erythropoiesis remains the principal therapeutic challenge for patients with more indolent subtypes, driven by a complex interplay between genetic abnormalities intrinsic to the MDS clone and senescence dependent inflammatory signals within the bone marrow (BM) microenvironment. Although three agents are approved for the treatment of MDS in the United States (US), lenalidomide (LEN) represents the only targeted therapeutic. Treatment with LEN yields sustained red blood cell transfusion independence accompanied by partial or complete resolution of cytogenetic abnormalities in the majority of patients with a chromosome 5q deletion (del(5q)), whereas only a minority of patients with non-del5q MDS achieve a meaningful response, infrequently accompanied by cytogenetic improvement. Although responses in patients with del5q MDS are relatively durable, lasting a median of 2.5 years, resistance emerges over time with resumption of transfusion dependence.

US 2015/224083 A1 discloses methods for treating cancer in a patient, comprising administration of a therapeutically effective regimen of cantharidin or a cantharidin analog to a patient in need thereof. Furthermore, US 2015/045373 A1 relates to compounds which may be used for the treatment of tumors. Moreover, WO 2016/014783 A1 discloses methods for treating a meylodysplastic syndrome in a subject that involves administering to the subject a therapeutically effective amount of a protein phosphatase 2A inhibitor.

### Summary of the Invention

This invention provides a compound for use in treating myelodysplastic syndrome in a human subject afflicted therewith, the use comprising administering to the subject an amount from 0.1 mg/m² to 5 mg/m² of a compound having the structure 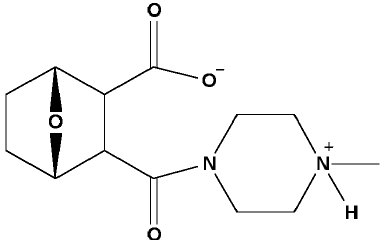 or a salt, zwitterion, or ester thereof, as defined in claim 1 with preferred embodiments being set out in the dependent claims.

Any references in the description to methods of treatment refer to the compounds, pharmaceutical compositions and medicaments of the present invention for use in a method for treatment of a human subject by therapy.

The scope of the present invention is defined in the claims; any other disclosure in the present description is provided for referential purposes, only.

### Detailed Description of the Invention

This invention provides a compound for use in treating myelodysplastic syndrome in a human subject afflicted therewith, the use comprising administering to the subject an amount from 0.1 mg/m² to 5 mg/m² of a compound having the structure 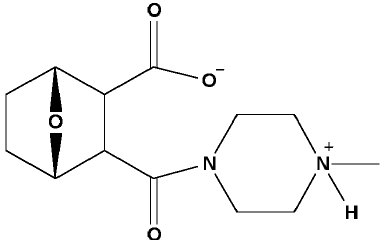 or a salt, zwitterion, or ester thereof, as defined in claim 1 with preferred embodiments being set out in the dependent claims.

In some embodiments, the amount of the compound administered is 0.25 mg/m² to 2.5 mg/m².

In some embodiments, the amount of the compound administered is 2.5 mg/m² to 5 mg/m².

In some embodiments, the amount of the compound administered is 3 mg/m² to 4.5 mg/m²

In some embodiments, the amount of the compound administered is 0.25 mg/m², 0.5 mg/m², 0.83 mg/m², 1.25 mg/m², 1.75 mg/m² or 2.33 mg/m² .

In some embodiments, the amount of the compound administered is about 0.25 mg/m², 0.5 mg/m², 0.75 mg/m², 1.0 mg/m², 1.25 mg/m², 1.5 mg/m², 1.75 mg/m², 2.0 mg/m², 2.25 mg/m², 2.5 mg/m² or 2.75 mg/m². In some embodiments, the amount of the compound administered is about 3 mg/m², 3.25 mg/m², 3.5 mg/m², 3.75 mg/m², 4 mg/m², 4.25 mg/m² or 4.5 mg/m².

In some embodiments, the amount of the compound administered is 0.83 mg/m² to 2.33 mg/m².

In some embodiments, the amount of the compound administered is about 0.83 mg/m², 1.25 mg/m², 1.75 mg/m², or 2.33 mg/m².

In some embodiments, the amount of the compound is administered once daily, weekly or monthly.

In some embodiments, the amount of the compound is administered once daily for a three day period.

In some embodiments, the amount of the compound is administered three times per week.

In some embodiments, the amount of the compound is administered on three separate days during a seven day period.

In some embodiments, the amount of the compound is administered on three separate days during a twenty-one day treatment cycle.

In some embodiments, the amount of the compound is administered on three separate days during week 1 of a twenty-one day treatment cycle.

21 In some embodiments, the amount of the compound is administered on days 1, 2 and 3 of a twenty-one day treatment cycle.

In some embodiments, the amount of the compound is administered on days 1, 2 and 3 of a twenty-one day treatment cycle and the cycle is repeated one or more times.

In some embodiments, the amount of the compound is administered on days 1, 2 and 3 of a twenty-one day treatment cycle and the cycle is repeated one or more times.

In some embodiments, the amount of the compound is administered on days 1, 2 and 3 of a twenty-one day treatment cycle and the cycle is repeated two or more times.

In some embodiments, the amount of the compound is administered on days 1, 2 and 3 of a twenty-one day treatment cycle and the cycle is repeated three or more times.

In some embodiments, the amount of the compound is administered on days 1, 2 and 3 of a twenty-one day treatment cycle and the cycle is repeated four or more times.

In some embodiments, the amount of the compound is administered on days 1, 2 and 3 of a twenty-one day treatment cycle and the cycle is repeated five or more times.

In some embodiments, the amount of the compound is administered on days 1, 2 and 3 of a twenty-one day treatment cycle and the cycle is repeated six or more times.

In some embodiments, the amount of the compound is administered on days 1, 2 and 3 of a twenty-one day treatment cycle and the cycle is repeated between 1 to 10 times.

In some embodiments, the compound is added to an amount of normal saline (0.9%) prior to administration to the subject.

In some embodiments, the compound is added to 500 mL of normal saline (0.9%) prior to administration to the subject.

In some embodiments, the compound is administered to the subject by intravenous infusion over 1 to 3 hours.

In some embodiments, the compound is administered to the subject by intravenous infusion over 2 hours.

Disclosed is a compound for use in treating MDS in a subject by administering to the subject a therapeutically effective amount of the disclosed pharmaceutical composition. The myelodysplastic syndromes (MDS) are hematological (blood-related) medical conditions with ineffective production (or dysplasia) of the myeloid class of blood cells. In some cases, the MDS patient has a chromosome 5q deletion (del(5q)). However, in other cases, the patient has non-del(5q) MDS.

In some embodiments, the subject afflicted with MDS has a chromosome 5q deletion (del(5q)).

In some embodiments, the subject afflicted with MDS with a chromosome 5q deletion has previously undergone failed prior treatment with at least 2 cycles of lenalidomide.

In some embodiments, the subject afflicted with MDS does not have a chromosome 5q deletion.

In some embodiments, the subject afflicted with non-del(5q) MDS has previously undergone failed prior treatment with at least 2 cycles of lenalidomide.

In some embodiments, the subject afflicted with non-del(5q) MDS has previously undergone failed prior treatment with at least 2 cycles of azacitidine.

In some embodiments, the subject afflicted with non-del(5q) MDS has previously undergone failed prior treatment with at least 2 cycles of decitabine.

In some embodiments, the compound is added to an amount of normal saline (0.9%) prior to administration to the subject.

In some embodiments, the compound is added to 500 mL of normal saline (0.9%) prior to administration to the subject.

In some embodiments, the compound is administered to the subject by intravenous infusion over 1 to 3 hours.

In some embodiments, the compound is administered to the subject by intravenous infusion over 2 hours.

In some embodiments, the treating comprises achievement of hematological improvement in the human subject.

In some embodiments, the hematological improvement comprises one or more of erythroid response, platelet response, or neutrophil response.

In some embodiments, the erythroid response comprises an Hgb increase by ≥ 1.5 g/dL, and there is a relevant reduction of units of RBC transfusions by an absolute number of at least 4 RBC transfusions/8 weeks compared with the pretreatment transfusion number in the previous 8 weeks, wherein only RBC transfusions given for a Hgb of ≤ 9.0 g/dL pretreatment will count in the RBC transfusion evaluation.

In some embodiments, the platelet response comprises an absolute increase of ≥ 30 x 10⁹/L platelets for subjects starting with > 20 x 10⁹/L platelets, or an increase from < 20 x 10⁹/L platelets to > 20 x 10⁹/L platelets, wherein the increase is by a proportion of at least 100%.

In some embodiments, the neutrophil response comprises at least a 100% increase in neutrophils, wherein the increase is an absolute increase of > 0.5 x 10⁹/L.

In some embodiments, the treating comprises achievement of a cytogenic response in the human subject.

In some embodiments, the cytogenic response comprises a complete response.

In some embodiments, the complete response comprises a disappearance of the chromosomal abnormality without appearance of new abnormalities.

In some embodiments, the cytogenic response comprises a partial response, wherein the partial response comprises at least a 50% reduction of the chromosomal abnormality.

In some embodiments, the treating comprises a complete remission of MDS in the human subject.

In some embodiments, the complete remission comprises achievement of ≤ 5% myeloblasts in the bone marrow with normal maturation of all cell lines, and achievement of hemoglobin ≥ 11 g/dL, platelets ≥ 100 x 10⁹/L, neutrophils ≥ 1.0 x 10⁹/L, and 0% myeloblasts in peripheral blood.

In some embodiments the treating comprises a partial remission of MDS in the human subject.

In some embodiments, the partial remission comprises achievement of a decrease of myeloblasts in the bone marrow of ≥ 50% over pretreatment with normal maturation of all cell lines, wherein the level of myeloblasts in the bone marrow is > 5%, and achievement of hemoglobin ≥ 11 g/dL, platelets ≥ 100 x 10⁹/L, neutrophils ≥ 1.0 x 10⁹/L, and 0% blasts in peripheral blood.

In some embodiments, the treating comprises marrow complete remission of MDS in the human subject.

In some embodiments, the marrow complete remission comprises achievement of a decrease of myeloblasts in the bone marrow of ≥ 50% over pretreatment, wherein the level of myeloblasts in the bone marrow is ≤ 5%.

In some embodiments, the treating comprises stabilization of MDS in the human subject.

In some embodiments, the stabilization of MDS comprises failure to achieve a decrease of myeloblasts of ≥ 50% over pretreatment, failure to achieve ≤ 5% myeloblasts, or failure to achieve normal maturation of all cell lines, in the bone marrow, or failure to achieve hemoglobin ≥ 11 g/dL, platelets ≥ 100 x 10⁹/L, neutrophils ≥ 1.0 x 10⁹/L, or 0% blasts in peripheral blood, and wherein the human subject exhibits no evidence of progression of the disease for > 8 weeks.

This invention also provides the compound for use in treating a subject suffering from myelodysplastic syndrome, wherein the compound is administered to the subject in an amount from 0.1 mg/m² to 5 mg/m².

This disclosure also provides for the use of the compound in the manufacture of a medicament for the treatment of myelodysplastic syndrome, wherein the medicament is administered to a subject suffering from myelodysplastic syndrome in an amount from 0.1 mg/m² to 5 mg/m².

This disclosure also provides a medicament comprising the compound for use in treating a patient who is suffering from myelodysplastic sydrome, wherein the medicament is to be administered in an amount from 0.1 mg/m² to 5 mg/m².

This disclosure also provides a treating agent for myelodysplastic syndrome in a patient comprising the compound as an active ingredient, wherein the compound is to be administered in an amount from 0.1 mg/m² to 5 mg/m², and wherein the patient is suffering from myelodysplastic syndrome.

LB-100 (3-{4methylpiperazine-carbonyl}-7-oxalobicyclo[2.2.1]heptane-2-carboxylic acid; NSC D753810), is a small molecule (MW 268) having the structure: 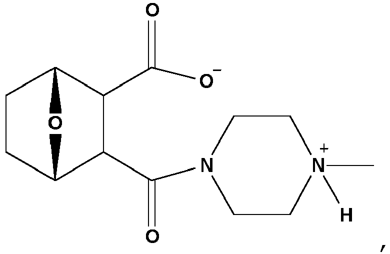 which may also be represented by the structure:

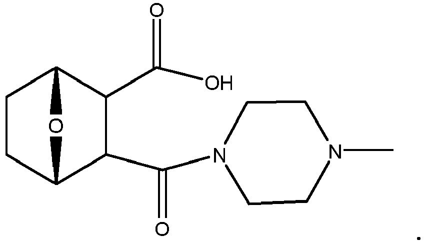

LB-100 inhibits PP2A about 80 fold more efficiently than protein phosphatase 1 (PP1). LB-100 is a synthetic derivative of cantharadin, a demethylated homolog of cantharadin (extract of beetle juice), with relative specificity *in vitro* and *in vivo* and acceptable toxicity (Hart, M.E. et al., 2004; Lu, J. et al., 2009; Zhuang, Z. et al., 2009; Zhang, C. et al., 2010). LB-100 was shown to increase Akt phosphorylation and decrease p53 expression in malignant glioma cells and xenografts (Lu, J. et al., 2009). LB-100 blocked cell cycle arrest and led to chemotherapy sensitization to temozolomide and doxorubicin (Lu, J. et al., 2009; Zhang, C. et al., 2010). LB-100 was also shown to induce tumor differentiation and/or cell death in glioblastoma multiforme (Lu, J. et al., 2010). LB-100 has a phase 1 clinical trial as a chemotherapy sensitizer in solid tumors (NCT01837667) (Vincent M. Chung, A.S.M., John Kovach, 2014).

Also disclosed is a pharmaceutical composition comprising the disclosed molecule in a pharmaceutically acceptable carrier. Pharmaceutical carriers are known to those skilled in the art. These most typically would be standard carriers for administration of drugs to humans, including solutions such as sterile water, saline, and buffered solutions at physiological pH. For example, suitable carriers and their formulations are described in Remington: The Science and Practice of Pharmacy (21 ed.) ed. PP. Gerbino, Lippincott Williams & Wilkins, Philadelphia, PA. 2005. Typically, an appropriate amount of a pharmaceutically-acceptable salt is used in the formulation to render the formulation isotonic. Examples of the pharmaceutically-acceptable carrier include, saline, Ringer's solution and dextrose solution. The pH of the solution is preferably from about 5 to about 8, and more preferably from about 7 to about 7.5. The solution should be RNAse free. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of composition being administered.

Pharmaceutical compositions may include carriers, thickeners, diluents, buffers, preservatives, and surface active agents in addition to the molecule of choice. Pharmaceutical compositions may also include one or more active ingredients such as antimicrobial agents, anti-inflammatory agents, and anesthetics.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, and electrolyte replenishers (such as those based on Ringer's dextrose). Preservatives and other additives may also be present such as, for example, antimicrobials, anti-oxidants, chelating agents, and inert gases.

Some of the compositions may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

The disclosed compositions, including pharmaceutical composition, may be administered in a number of ways depending on whether local or systemic treatment is desired, and on the area to be treated. For example, the disclosed compositions can be administered intravenously, intraperitoneally, intramuscularly, subcutaneously, intracavity, or transdermally. The compositions may be administered orally, parenterally (e.g., intravenously), by intramuscular injection, by intraperitoneal injection, transdermally, extracorporeally, ophthalmically, vaginally, rectally, intranasally, topically or the like, including topical intranasal administration or administration by inhalant.

Parenteral administration of the composition, if used, is generally characterized by injection. Injectables can be prepared in conventional forms, either as liquid solutions or suspensions, solid forms suitable for solution of suspension in liquid prior to injection, or as emulsions. A revised approach for parenteral administration involves use of a slow release or sustained release system such that a constant dosage is maintained.

The compositions disclosed herein may be administered prophylactically to patients or subjects who are at risk for MDS. Thus, the use can further comprise identifying a subject at risk for MDS prior to administration of the herein disclosed compositions.

The exact amount of the compositions required will vary from subject to subject, depending on the species, age, weight and general condition of the subject, the severity of the allergic disorder being treated, the particular nucleic acid or vector used, and its mode of administration. Thus, it is not possible to specify an exact amount for every composition. However, an appropriate amount can be determined by one of ordinary skill in the art using only routine experimentation given the teachings herein. For example, effective dosages and schedules for administering the compositions may be determined empirically, and making such determinations is within the skill in the art. The dosage ranges for the administration of the compositions are those large enough to produce the desired effect in which the symptoms disorder are affected. The dosage should not be so large as to cause adverse side effects, such as unwanted cross-reactions, and anaphylactic reactions. Generally, the dosage will vary with the age, condition, sex and extent of the disease in the patient, route of administration, or whether other drugs are included in the regimen, and can be determined by one of skill in the art. The dosage can be adjusted by the individual physician in the event of any counterindications. Dosage can vary, and can be administered in one or more dose administrations daily, for one or several days. Guidance can be found in the literature for appropriate dosages for given classes of pharmaceutical products. A typical daily dosage of the disclosed composition used alone might range from about 1 µg/kg to up to 100 mg/kg of body weight or more per day, depending on the factors mentioned above.

The present disclosure also provides a package comprising:
1) a pharmaceutical composition comprising an amount of LB-100 and a pharmaceutically acceptable carrier; and
2) instructions for use of the pharmaceutical composition to treat MDS.

The present disclosure provides a pharmaceutical composition comprising LB-100 and at least one pharmaceutically acceptable carrier for use in treating MDS.

In some embodiments, the pharmaceutical composition wherein the pharmaceutically acceptable carrier comprises a liposome.

In some embodiments, the pharmaceutical composition wherein the compound is contained in a liposome or microsphere.

### Definitions

As used herein, "treatment of the disease" or "treating", encompasses inducing prevention, inhibition, regression, remission or stabilization of the disease or a symptom or condition associated with the disease.

As used herein, "failed prior treatment" with a pharmaceutical compound is defined as no response to treatment, loss of response at any time point, or progressive disease/intolerance to therapy.

As used herein, "inhibition" of disease progression or disease complication in a subject means preventing or reducing the disease progression and/or disease complication in the subject.

As used herein, "administering" an agent may be performed using any of the various methods or delivery systems well known to those skilled in the art. The administering can be performed, for example, orally, parenterally, intraperitoneally, intravenously, intraarterially, transdermally, sublingually, intramuscularly, rectally, transbuccally, intranasally, liposomally, via inhalation, vaginally, intraoccularly, via local delivery, subcutaneously, intraadiposally, intraarticularly, intrathecally, into a cerebral ventricle, intraventicularly, intratumorally, into cerebral parenchyma or intraparenchchymally.

The following delivery systems, which employ a number of routinely used pharmaceutical carriers, may be used but are only representative of the many possible systems envisioned for administering compositions in accordance with the disclosure. Injectable drug delivery systems include solutions, suspensions, gels, microspheres and polymeric injectables, and can comprise excipients such as solubility-altering agents (e.g., ethanol, propylene glycol and sucrose) and polymers (e.g., polycaprylactones and PLGA's).

Other injectable drug delivery systems include solutions, suspensions, gels. Oral delivery systems include tablets and capsules. These can contain excipients such as binders (e.g., hydroxypropylmethylcellulose, polyvinyl pyrilodone, other cellulosic materials and starch), diluents (e.g., lactose and other sugars, starch, dicalcium phosphate and cellulosic materials), disintegrating agents (e.g., starch polymers and cellulosic materials) and lubricating agents (e.g., stearates and talc).

Implantable systems include rods and discs, and can contain excipients such as PLGA and polycaprylactone.

Oral delivery systems include tablets and capsules. These can contain excipients such as binders (e.g., hydroxypropylmethylcellulose, polyvinyl pyrilodone, other cellulosic materials and starch), diluents (e.g., lactose and other sugars, starch, dicalcium phosphate and cellulosic materials), disintegrating agents (e.g., starch polymers and cellulosic materials) and lubricating agents (e.g., stearates and talc).

Transmucosal delivery systems include patches, tablets, suppositories, pessaries, gels and creams, and can contain excipients such as solubilizers and enhancers (e.g., propylene glycol, bile salts and amino acids), and other vehicles (e.g., polyethylene glycol, fatty acid esters and derivatives, and hydrophilic polymers such as hydroxypropylmethylcellulose and hyaluronic acid).

Dermal delivery systems include, for example, aqueous and nonaqueous gels, creams, multiple emulsions, microemulsions, liposomes, ointments, aqueous and nonaqueous solutions, lotions, aerosols, hydrocarbon bases and powders, and can contain excipients such as solubilizers, permeation enhancers (e.g., fatty acids, fatty acid esters, fatty alcohols and amino acids), and hydrophilic polymers (e.g., polycarbophil and polyvinylpyrolidone). In one embodiment, the pharmaceutically acceptable carrier is a liposome or a transdermal enhancer.

Solutions, suspensions and powders for reconstitutable delivery systems include vehicles such as suspending agents (e.g., gums, zanthans, cellulosics and sugars), humectants (e.g., sorbitol), solubilizers (e.g., ethanol, water, PEG and propylene glycol), surfactants (e.g., sodium lauryl sulfate, Spans, Tweens, and cetyl pyridine), preservatives and antioxidants (e.g., parabens, vitamins E and C, and ascorbic acid), anti-caking agents, coating agents, and chelating agents (e.g., EDTA).

As used herein, "pharmaceutically acceptable carrier" refers to a carrier or excipient that is suitable for use with humans and/or animals without undue adverse side effects (such as toxicity, irritation, and allergic response) commensurate with a reasonable benefit/risk ratio. It can be a pharmaceutically acceptable solvent, suspending agent or vehicle, for delivering the instant compounds to the subject.

The compounds for use of the present invention may be in a salt form. As used herein, a "salt" is a salt of the instant compounds which has been modified by making acid or base salts of the compounds. In the case of compounds used to treat an infection or disease, the salt is pharmaceutically acceptable. Examples of pharmaceutically acceptable salts include, mineral or organic acid salts of basic residues such as amines; alkali or organic salts of acidic residues such as phenols. The salts can be made using an organic or inorganic acid. Such acid salts are chlorides, bromides, sulfates, nitrates, phosphates, sulfonates, formates, tartrates, maleates, malates, citrates, benzoates, salicylates, and ascorbates. Phenolate salts are the alkaline earth metal salts, sodium, potassium or lithium. The term "pharmaceutically acceptable salt" in this respect, refers to the relatively nontoxic, inorganic and organic acid or base addition salts of compounds for use of the present invention. These salts can be prepared in situ during the final isolation and purification of the compounds for use of the invention, or by separately reacting a purified compound for use of the invention in its free base or free acid form with a suitable organic or inorganic acid or base, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts. (See, e.g., Berge et al. (1977) "Pharmaceutical Salts", J. Pharm. Sci. 66:1-19) .

The present invention includes esters or pharmaceutically acceptable esters of the compounds for use in the present use. The term "ester" includes, a compound containing the R-CO-OR' group. The "R-CO-O" portion may be derived from the parent compound of the compounds for use of the present invention. The "R'" portion includes, alkyl, alkenyl, alkynyl, heteroalkyl, aryl, and carboxy alkyl groups.

The present disclosure includes pharmaceutically acceptable prodrug esters of the compounds for use in the present use. Pharmaceutically acceptable prodrug esters of the compounds for use of the present invention are ester derivatives which are convertible by solvolysis or under physiological conditions to the free carboxylic acids of the parent compound. An example of a prodrug is an alkly ester which is cleaved *in vivo* to yield the compound of interest.

As used herein, an "amount" or "dose" of an agent measured in milligrams refers to the milligrams of agent present in a drug product, regardless of the form of the drug product.

As used herein, the term "therapeutically effective amount" or "effective amount" refers to the quantity of a component that is sufficient to yield a desired therapeutic response without undue adverse side effects (such as toxicity, irritation, or allergic response) commensurate with a reasonable benefit/risk ratio when used in the manner of this invention. The specific effective amount will vary with such factors as the particular condition being treated, the physical condition of the patient, the type of mammal being treated, the duration of the treatment, the nature of concurrent therapy (if any), and the specific formulations employed and the structure of the compounds or its derivatives.

Where a range is given in the specification it is understood that the range includes all integers and 0.1 units within that range, and any sub-range thereof. For example, a range of 77 to 90% is a disclosure of 77, 78, 79, 80, and 81% etc.

As used herein, "about" with regard to a stated number encompasses a range of +one percent to -one percent of the stated value. By way of example, about 100 mg/m² therefore includes 99, 99.1, 99.2, 99.3, 99.4, 99.5, 99.6, 99.7, 99.8, 99.9, 100, 100.1, 100.2, 100.3, 100.4, 100.5, 100.6, 100.7, 100.8, 100.9 and 101 mg/m². Accordingly, about 100 mg/m² includes, in an embodiment, 100 mg/m².

It is understood that where a parameter range is provided, all integers within that range, and tenths thereof, are also provided by the invention. For example, "0.2-5 mg/m²" is a disclosure of 0.2 mg/m², 0.3 mg/m², 0.4 mg/m², 0.5 mg/m², 0.6 mg/m² etc. up to 5.0 mg/m².

For the foregoing embodiments, each embodiment disclosed herein is contemplated as being applicable to each of the other disclosed embodiment.

This invention will be better understood by reference to the Experimental Details which follow.

### Examples

### Abbreviations

AE Adverse Event
CFR Code of Federal Regulations
CR Complete Response
CRF Case Report Form
CTCAE Common Terminology Criteria for Adverse Events
DLT Dose Limiting Toxicity
ECOG Eastern Cooperative Oncology Group
FDA Food and Drug Administration
GLP Good Laboratory Practice
HIV Human Immunodeficiency Virus
HNSTD Highest Non-Severely Toxic Dose
IRB/IEC Institutional Review Board/Independent Ethics Committee
ITT Intent-to-Treat
IV Intravenous
PD Progressive Disease
MTD Maximum Tolerated Dose
NCI National Cancer Institute
NIH National Institutes of Health
NOAEL No Observed Adverse Effect Level
NOEL No Observed Effect Level
PBS Phosphate Buffered Saline
PK Pharmacokinetics
PR Partial Response
SAE Serious Adverse Event
SD Stable Disease
ULN Upper Limit of Normal
WBC White Blood Cell

### Example 1

### Toxicology in Rats:

In a non-GLP dose ranging study in male Fischer rats, LB-100 was administered by daily intravenous (IV) infusion at 0.5, 0.75 and 1.5 mg/kg/day for 4 consecutive days. There were no unscheduled deaths in any of the treatment groups. A no-observed-adverse-effect-level (NOAEL) was not established in this study. The MTD was 0.75 mg/kg/day (about 4.5 mg/m²) when administered IV daily for 4 days. At 1.5 mg/kg/day, clinical observations included blood in urine (Day 4), lethargy (Days 3 and 4), and hind limb paresis (Day 4). At 1.5 mg/kg/day, adverse effects in kidney (nephrosis) in the distal convoluted tubules were seen in 3 of 3 rats; in the 0.75 mg/kg/day group, nephrosis was mild, and in the 0.5 mg/kg/day group, nephrosis was minimal. Primary clinical signs of blood in the urine and clinical chemistry findings of increased blood urea nitrogen and creatinine supported kidney and urinary bladder as target organs of toxicity. The transient hind limb paresis observed at the 1.5 mg/kg/day dose level had no histopathology correlates that would explain the paresis. Heart toxicity (epicardial hyperplasia with inflammation primarily on the epicardium of the atria) was observed in the 0.75 and 1.5 mg/kg/day groups. The hyperplasia was accompanied by subepicardial accumulation of mononuclear cells and eosinophils. One rat in the 1.5 mg/kg/day group had a large focus of inflammation with eosinophils associated with the aorta. Kidney, heart, femoral bone, liver and urinary bladder toxicity appeared to be dose-limiting toxicities in rats treated with LB-100 when administered IV once per day for 4 consecutive days.

In the GLP repeat-dose study in rats, LB-100 administered via daily intravenous (slow bolus) injection for 5 consecutive days to male and female Sprague Dawley rats at dose levels of 0.5, 0.75, and 1.25 mg/kg/day resulted in adverse, test article-related nephrosis of the kidneys in the 0.75 and 1.25 mg/kg/day group males and females, which persisted or progressed in the 0.75 and 1.25 mg/kg/day group males at the recovery necropsy. Test article-related effects on urinalysis parameters were observed in all treatment groups and included an increase in incidence and severity of urine occult blood in 0.5 mg/kg/day group males and 0.75 and 1.25 mg/kg/day group males and females, urine protein in 1.25 mg/kg/day females, and increase in microscopic observations of leukocytes in males and females of the 1.25 mg/kg/day group, and in one female in both the 0.5 and 0.75 mg/kg/day group on Day 5. These changes were reversible. LB-100 administration resulted in subacute subepicardial inflammation and/or mesothelial hypertrophy in the atria of males at ≥ 0.5 mg/kg/day and at 1.25 mg/kg/day in the females at the primary necropsy and was considered adverse in one 1.25 mg/kg/day group male. Minimal to mild subacute inflammation was observed in the epicardium and subepicardium of the left and/or right atrium of the heart in the 0.5, 0.75, and 1.25 mg/kg/day group males and the 1.25 mg/kg/day group females. One male in the 1.25 mg/kg/day group had mild subacute inflammation that was accompanied by minimal fibroplasia (plump fibroblasts) in the right atrium. Inflammation was often accompanied by mesothelial hypertrophy. There was a higher incidence of mesothelial hypertrophy in the 1.25 mg/kg/day group females when compared to the control group. Based on these findings, the severely toxic dose in 10% of the animals (STD 10) for this study was determined as 0.75 mg/kg/day. This dose corresponded to AUCₗₐₛₜ values of 596 and 691 ng•h/mL and C₀ values of 1804 and 2347 ng/mL for males and females, respectively, on study Day 4. LB-100 has shown *in vitro* and *in vivo* activity as a single agent as well as potentiating the activity of cytotoxic agents including temozolomide, doxorubicin, docetaxel and ionizing radiation in vivo. LB-100 is active in combination with temozolomide or doxorubicin.

### Example 2

### Toxicity in Dogs:

In a non-GLP dose ranging study, LB-100 administered intravenously (slow bolus push) to beagle dogs at dose levels of 0.1, 0.25, 0.5, and 1.0 mg/kg given every 4 days x 4 doses (on study days 0, 4, 8, and 12) resulted in a no observed-effect level (NOEL) of 0.25 mg/kg. There were no LB-100-related effects on survival. A possible test article-related clinical observation of intermittent tremors was noted in one female on study Day 13 following administration of LB-100 at 1.0 mg/kg. At dose levels of 0.5 and 1.0 mg/kg, lower body weight gains and food consumption were noted in females.

In the GLP repeat dose dog study, LB-100 was administered by intravenous injection (slow bolus push) at dose levels of 0.15, 0.30, and 0.75 mg/kg daily for 5 consecutive days. Test article-related lethality was observed in 2 of 10 animals in the 0.75 mg/kg/day group, a male and a female were found dead prior to administration of the fourth scheduled dose. The dosage level was reduced to 0.50 mg/kg/day for the 4th and 5th doses (study Days 3 and 4). Both animals dying after the 3rd dose at 0.75 mg/kg/day had similar test article-related macroscopic and microscopic findings affecting the gastrointestinal tract, kidneys, injection sites (hemorrhage), spleen, larynx, lungs (including acute inflammation) and/or liver. Both animals had experienced emesis, decreased defecation, yellow and red mucoid feces, and red diarrhea; these changes were also observed in animals treated at the 0.3 mg/kg/day dose level.

Although the most noteworthy findings (mitotic figures and single cell necrosis of the renal tubular epithelial cells from the outer medulla and cortex) could be associated with altered renal function, these findings were not considered fatal lesions; therefore, the cause of death for each animal was considered undetermined but directly attributed to test article administration. Note: a dose of 0.75 mg/kg in the dog (average weight of 9 kg and BSA of 0.5 m2) is about 13.8 mg/m2 or more than twice the MTD in the rat. This highest dose was selected because the dose range study in the dog revealed almost no signs of toxicity following a single dose of 1.0 mg/kg (approximately 18 mg/m2) in the dose ranging study. All other animals survived to the scheduled primary (study Day 5) and recovery (study Day 29) necropsies including the dogs receiving 0.75 mg/kg daily for 3 days and 0.5 mg/kg for doses 4 and 5. Test article-related histological changes at the Day 5 necropsy included erosion and focal hemorrhage within the gastrointestinal tract in the 0.75/0.5 mg/kg/day dose group. Single cell necrosis was observed throughout the gastrointestinal tract.

These changes were reported as resolved in the recovery period. There were no ophthalmic findings or changes in electrocardiography parameters and blood pressures associated with test article administration in any treatment group.

During the recovery period, all surviving animals had body weight gains indicative of recovery, and the majority of the other observed clinical signs resolved within the first few days of the recovery period. At the primary necropsy (Day 5), test article-related macroscopic findings consisted of dark red discoloration of the kidneys, small spleens, and red discoloration (reddened mucosa or dark red areas) of various segments of the gastrointestinal tract in the 0.75/0.50 mg/kg/day group males and females.

At the recovery necropsy (Day 29), no test article-related macroscopic findings were observed. The primary cause of small spleen size appeared to be due to less blood in the red pulp. Mild or moderate single cell (lymphoid) necrosis was seen in spleens microscopically. Test article-related effects on hematology and coagulation parameters at the Day 5 evaluation included higher red blood cell mass (red blood cell count, hemoglobin, and hematocrit), lower platelet counts, and prolonged activated partial thromboplastin time values in the animals of the 0.75/0.50 mg/kg/day group. In this group, lower platelet counts were statistically significantly lower only in the males, with the group mean level lower than the historical control group mean level. Lower platelet count in a female was not statistically significant but was considered test-article related. At the Day 29 evaluation, there were no residual effects of test article administration on hematology or coagulation parameters. Test article-related changes in urinalysis parameters observed at the Day 5 evaluation included lower specific gravity, higher urine volume, and increased presence of blood in the 0.75/0.50 mg/kg/day groups. At Day 29, no test article-related changes in urinalysis parameters were present.

Multilead (I, II, III, aVR, aVL, aVF, and V2) ECGs were recorded for all animals prior to randomization (Day -8) and for all surviving animals on Day 4 (recorded approximately 2 to 4 hours following dose administration) and Day 27. All the ECGs were qualitatively and quantitatively interpreted and within normal limits. No test article-related effects attributable to the test article administration were found at any dose level based on comparison of pretest and post-dosing group mean values and control values. No abnormalities in rhythm were found.

Blood pressure (systolic, diastolic, and mean arterial pressure) data were recorded for all animals once during the pretest period (Day -8) and for all surviving animals on study Day 4 (recorded approximately 2 to 4 hours following dose administration) and Day 27. Blood pressure was unaffected by test article administration. There were no statistically significant differences at the Days 4 and 27 evaluations when the control and test article-treated groups were compared.

In conclusion, administration of LB-100 via daily intravenous (slow bolus) injection for 5 consecutive days to male and female beagle dogs was well tolerated at the dosage level of 0.15 mg/kg/day. At dosage levels of 0.30 and 0.75/0.50 mg/kg/day, administration of LB-100 resulted in adverse clinical observations, lower body weights, and histological findings (congestion and nephrosis in kidneys, increased mitoses and single cell necrosis in liver, lymphoid depletion and single cell necrosis in thymus, and/or erosion and/or hemorrhage in stomach or intestines) correlating with effects on clinical pathology, organ weight, and/or macroscopic findings during the dosing period. Persistent adverse test article related histological changes in the kidneys were observed in the 0.30 and 0.75/0.50 mg/kg/day group males and females at the Day 29 recovery necropsy. These changes were more indicative of a progression towards chronicity rather than recovery. In addition, lethality was observed at 0.75 mg/kg/day. Therefore, the Highest Non-Severely Toxic Dose (HNSTD) was 0.15 mg/kg, which corresponded to an AUCₗₐₛₜ for LB-100 of 267 and 335 ng•h/mL on study day 4 for males and females, respectively.

### Example 3

### Current Clinical Studies:

There is a clinical trial with LB-100 as a chemotherapy sensitizer in solid tumors (NCT01837667). The trial observed no dose limiting toxicities on dose level 6 (2.33 mg/m²).

Specifically, there have been no cardiac or myelosuppressive toxicities observed. At dose level 6, plasma concentrations of LB-100 are greater than 1µM.

LB-100 is well tolerated in the phase 1 clinical trial. Renal changes were observed in GLP toxicity studies of rats and/or dogs given LB-100 at higher doses. In rats, these included nephrosis of the kidneys and increases in urine occult blood, urine protein, and microscopic observation of leukocytes; these changes were reversible. In rats, minimal to mild subacute inflammation was observed in the epicardium and subepicardium of the left and/or right atrium of the heart; inflammation was often accompanied by mesothelial hypertrophy. In dogs, changes in urinalysis parameters on Day 5 included lower specific gravity, higher urine volume and increased presence of blood at higher doses; at Day 29, no test article-related changes in urinalysis parameters were present. Other effects observed in dogs, generally at higher doses, included transient decreases in platelet counts that recovered by Day 29, gastrointestinal effects (including emesis, diarrhea, erosion, focal hemorrhage, and single cell necrosis) that generally resolved during the recovery period, changes in the lungs including acute inflammation, and hemorrhage at the injection site. No abnormalities were observed in ECGs or blood pressure in dogs administered LB-100 for 5 consecutive days.

Renal function of patients is closely monitored during the study. Urinalysis and evaluation of blood chemistry is done pre-study, at least weekly during the study, and at off-study. Patients are also monitored for the development of neurological symptoms, as transient hind limb paresis was reported in rats given LB-100 at higher doses.

### Study Endpoints:

### Primary endpoints:

- Phase 1b: in the first 2 cycles (6 weeks), the occurrence of DLTs, as defined below, graded according to the National Cancer Institute Common Terminology Criteria for Adverse Events (NCI CTCAE), Version 4.03.
- Phase 2: Achievement of hematological improvement (HI) and or cytogenetic response by the IWG 2006 criteria (see Tables 1 and 2). Patients who achieve such a response will be categorized as "responders" and the rest of the patients will be categorized as non-responders.

**Table 1: Response Criteria for Subjects with MDS and CMML According to the IWG 2006 Criteria**

| **ALTERING DISEASE NATURAL HISTORY** | |
|---|---|
| Complete remission (CR) | Bone marrow: ≤ 5% myeloblasts with normal maturation of all cell lines. |
| | Persistent dysplasia will be noted |
| | Peripheral blood: |
| | Hemoglobin ≥ 11 g/dL |
| | Platelets ≥ 100 x 10⁹/L |
| | Neutrophils ≥ 1.0 x 10⁹/L |
| | Blasts 0% |
| Partial remission (PR) | All CR criteria if abnormal before treatment, except: |
| | Bone marrow blasts decreased by ≥ 50% over pretreatment but still > 5% |
| | Cellularity and morphology not relevant |
| Marrow CR | Bone marrow ≤ 5% myeloblasts and decrease by ≥ 50% over pretreatment |
| | Peripheral blood: if HI responses, they will be noted in addition to marrow CR |
| Stable disease (SD) | Failure to achieve at least PR, but no evidence of progression for > 8 weeks |
| Failure | Death during treatment |
| | Disease progression characterized by worsening of cytopenias, increase in % of bone marrow blasts, or progression to a more advanced MDS FAB subtype than pretreatment |
| Disease Progression (PD) | For subjects with: |
| | Less than 5% blasts: ≥ 50% increase in blasts to > 5% blasts |
| | 5%-10% blasts: ≥ 50% increase in blasts to > 10% blasts |
| | 10%-20% blasts: ≥ 50% increase in blasts to > 20% blasts |
| | 20%-30% blasts: ≥ 50% increase in blasts to > 30% blasts |
| | Any of the following: |
| | At least 50% decrement from maximum remission/response levels in |
| | granulocytes or platelets |
| | Reduction in hemoglobin (Hgb) concentration by ≥ 2g/dL |
| | Transfusion dependence |

| **CYTOGENIC RESPONSE** | |
|---|---|
| Complete | Disappearance of the chromosomal abnormality without appearance of new ones |
| Partial | At least 50% reduction of the chromosomal abnormality |

| **HEMATOLOGICAL IMPROVEMENT (HI)** | |
|---|---|
| Erythroid response (HIE) (Pretreatment < 11 g/dL) | Hgb increase by ≥ 1.5 g/dL |
| | Relevant reduction of units of RBC transfusions by an absolute number of at least 4 RBC transfusions/8 weeks compared with the pretreatment transfusion number in the previous 8 weeks. Only RBC transfusions given for a Hgb of ≤ 9.0 g/dL pretreatment will count in the RBC transfusion evaluation |
| Platelet response (HI-P) (Pretreatment < 100 x 10⁹/L) | Absolute increase of ≥ 30 x 10⁹/L for subjects starting with > 20 x 10⁹/L |
| | Increase from < 20 x 10⁹/L to > 20 x 10⁹/L and by at least 100% |
| Neutrophil response (HI-N) (Pretreatment < 1.0 x 10⁹/L) | At least 100% increase and an absolute increase of > 0.5 x 10⁹/L |

**Table 2: Progression/Relapse Criteria for Subjects with MDS and CMML**

| **ALTERING DISEASE NATURAL HISTORY** | |
|---|---|
| Disease Progression (PD) | For subjects with: |
| | Less than 5% blasts: ≥ 50% increase in blasts to > 5% blasts |
| | 5%-10% blasts: ≥ 50% increase in blasts to > 10% blasts |
| | 10%-20% blasts: ≥ 50% increase in blasts to > 20% blasts |
| | 20%-30% blasts: ≥ 50% increase in blasts to > 30% blasts |
| | Any of the following: |
| | At least 50% decrement from maximum remission/response levels ingranulocytes or platelets |
| | Reduction in hemoglobin (Hgb) concentration by ≥ 2g/dL |
| | Transfusion dependence |
| Disease transformation | Transformation to AML (30% or more blasts) |
| Relapse after CR or PR | At least one of the following: |
| | Return to pretreatment bone marrow blast % |
| | Decrement of ≥ 50% from maximum remission/response levels ingranulocytes or platelets |
| | Reduction in Hgb concentration by ≥ 1.5 g/dL or transfusion dependence |

| **HEMATOLOGICAL IMPROVEMENT (HI)** | |
|---|---|
| Progression/relapse after HI | At least one of the following: |
| | At least 50% decrement from maximum response levels in granulocytes or platelets |
| | Reduction in Hgb concentration by ≥ 1.5 g/dL |
| | Transfusion dependence |

### Secondary endpoints:

- Plasma concentrations within Phase 1b cohort only.
- The response of MDS patients with del (5q) who achieve HI and/or cytogenic response.
- Duration of response defined as the time from achieve of HI and/or cytogenic response until progression of disease or death due to disease.
- Acute myeloid leukemia (AML) transformation according to World Health Organization (WHO) criteria (see Tables 1 and 2) .
- PP2A activity measured via Active PP2A assay kit in peripheral blood before and after LB-100 administration and assess downstream target inhibition in phosphorylation of PP2A substrates (e.g. CDC25C, MDM2, AKT) and p53 expression by immunohistochemistry (IHC) in bone marrow (BM) samples.
- Erythropoietin-induced STAT5 activation in erythroid progenitors as measured by flow cytometry.
- Determine recurrent gene mutations in ABL1, ASXL1, CBL, CEBPA, CSF3R, CUX1, DNMT3A, ETV6, EZH2, FLT3, IDH1, IDH2, IKZF1, JAK2, KIT, KRAS, MLL, MPL, MYD88, NPM1, NRAS, PHF6, RUNX1, SETBP1, SF3B1, SH2B3, SRSF2, TET2, TP53, U2AF1, WT1, and ZRSR2 at study entry and at best response/end of study and/or progression of disease.

### Selection of Patients:

### Inclusion Criteria:

1. Patient has signed the Informed Consent Form (ICF) and is able to comply with protocol requirements
2. Patient has adequate organ function as defined by the following laboratory values:
   a. Serum creatinine ≤ 2 x upper limit of normal (ULN)
   b. Total serum bilirubin < 1.5 x ULN or total bilirubin ≤ 3.0 x ULN with direct bilirubin within normal range in patients with well documented Gilbert's syndrome or hemolysis or who required regular blood transfusions
   c. Alanine aminotransferase (AST) and aspartate aminotransferase (ALT) < 3.0 x ULN
3. Age ≥18 years at the time of signing the informed consent form.
4. Documented diagnosis of MDS or MDS/MPN by World Health Organization (WHO) criteria that meets the IPSS criteria for low or int-1 risk.
5. For non-del(5q) patients, failed prior treatment with at least 2 cycles of azacitidine or decitabine or lenalidomide defined as no response to treatment, loss of response at any time point, or progressive disease/intolerance to therapy.
6. For del(5q) patients, failed prior treatment with at least 2 cycles of lenalidomide defined as no response to treatment, loss of response at any time point, or progressive disease/intolerance to therapy.
7. An Eastern Cooperative Oncology Group (ECOG) performance status score of 0, 1, or 2
8. Women of child-bearing potential and men must agree to use adequate contraception (hormonal or barrier method of birth control; abstinence; tubal ligation, partner's vasectomy) for at least 28 days prior to study entry and for the duration of study participation. Should a woman become pregnant or suspect she is pregnant while participating in this study, she should inform her treating physician immediately.

### Exclusion Criteria:

1. Patient has a known history of HIV infection (testing not mandatory).
2. Patient has any of the following cardiac abnormalities:
   a. symptomatic congestive heart failure
   b. myocardial infarction ≤ 6 months prior to enrolment
   c. unstable angina pectoris
   d. serious uncontrolled cardiac arrhythmia
3. Concomitant malignancies or previous malignancies with less than a 2-year disease free interval at the time of enrollment. Patients with adequately resected basal or squamous cell carcinoma of the skin, or adequately resected carcinoma in situ (i.e. cervix) may enroll irrespective of the time of diagnosis.
4. Use of chemotherapeutic agents or experimental agents (agents that are not commercially available) for the treatment of MDS within 14 days of the first day of study drug treatment. Growth factor support may be used for the short-term management of neutropenic infection. Stable doses of erythropoietin stimulating agents that were started >8 weeks from first LB-100 are allowed.
5. Pregnant women are excluded from this study because LB-100 has not been studied in pregnant subjects. Because there is an unknown but potential risk for adverse events in nursing infants secondary to treatment of the mother with LB-100, breastfeeding should be discontinued if the mother is treated with LB-100.

### Inclusion of Women and Minorities:

Both men and women and members of all races and ethnic groups are eligible.

### Study Design:

This study is a single institution, open-label, Phase 1b/2 clinical trial evaluating the toxicity and efficacy of intravenous LB-100 in lower risk MDS patients that is be conducted in 2 parts: a Phase 1 Dose Finding part followed by a Simon's two-stage Phase 2 design. A stopping rule is used to monitor for too many unacceptable adverse events, and patients are monitored for the transformation to acute myeloid leukemia as defined by WHO (see Tables 1 and 2). Eligible subjects must have a WHO diagnosis of MDS or MDS/MPN and meet IPSS criteria for low or int-1 risk disease and have previously failed a hypomethylating agent or lenalidomide (lenalidomide failure/intolerance mandatory for del(5q) patients). In the Dose Finding Phase, patients receive intravenous infusions of LB-100 over 15 minutes on days 1-3 of each 21 day cycle at escalating doses starting at Dose Level 1 (see Table 3). Patients are followed for at least 6 weeks (2 cycles) before the safety of each cohort can be fully assessed and decisions made for dose escalation in the next cohort. The MTD is defined as the dose level below which DLT is manifested in ≥33% of the patients or at dose level 2 if DLT is manifested in <33% of the patients.

**Table 3:**

| **Dose Levels for Treatment Part 1: LB-100 Single Agent Dose Level LB-100 (mg/m²)** | |
|---|---|
| **Dose Level** | **LB-100 (mg/m²)** |
| -2 | 0.83 |
| -1^{(a)} | 1.25 |
| 1 (starting dose) | 1.75 |
| 2 | 2.33 |
| ^{a} In the event that DLT is observed at Dose Level 1, subsequent patients will be enrolled in Dose Level -1. | |

Following completion of the Dose Finding Phase, a dose expansion is conducted, whereby patients are treated with LB-100 administered intravenously at the MTD using the 21 day cycle as determined from the Dose Escalation Phase. A Simon's two-stage design is applied, as follows: Stage 1: Enroll a total of 21 evaluable patients at the MTD (including patients who were enrolled during the Phase 1 part of the study). If there are 2 or fewer responders, as defined by the IWG 2006 criteria (HI and/or cytogenetic response), then the study is terminated early with the conclusion that the regimen does not warrant further investigation. If there are 3 or more responders, then enrollment is permitted to continue to Stage 2.

Stage 2: Enroll 20 more evaluable patients for a total of 41. If there are 6 or fewer responders, then there is insufficient evidence to support continued study of this treatment. If there are 7 or more responders, then there is sufficient evidence to support further study of LB-100 in Phase 3.

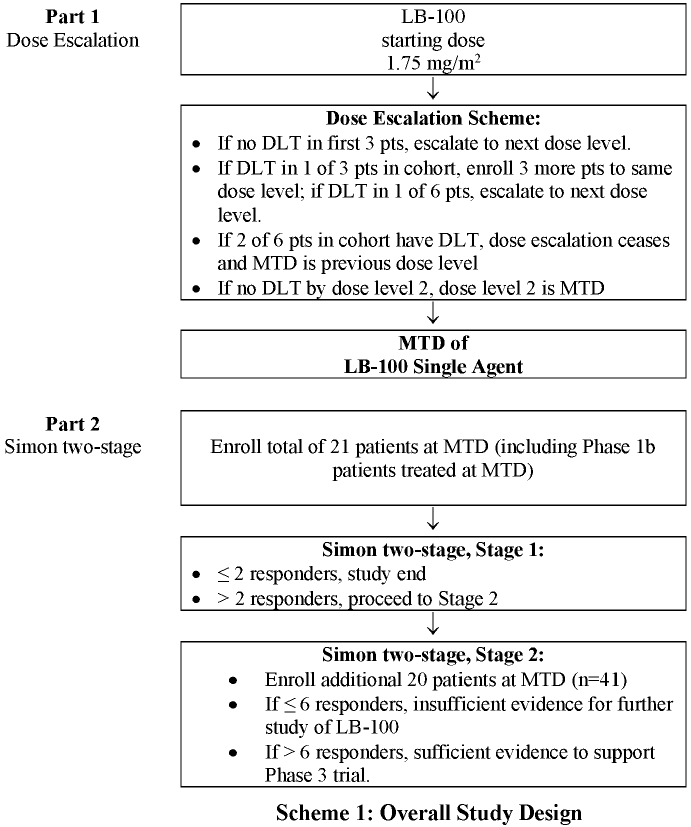

### Dose Limiting Toxicity

The NCI Common Terminology Criteria for Adverse Events (CTCAE) Version 4.03 will be used to grade toxicity. DLT is defined as any of the following adverse events occurring through the end of Cycle 2 of treatment and considered to be possibly, probably, or definitely related to study treatment:
1. Treatment related non-hematological CTCAE grade 3-4 toxicity except as follows:
   a. Grade 3 metabolic/electrolyte abnormalities that are not clinically significant, and are adequately controlled within 72 hours are not to be considered DLT.
   b. Grade 3 nausea/vomiting/diarrhea despite maximum medical therapy.

Patients with DLT during the DLT assessment period (through the end of Cycle 2) are taken off study and receive no further treatment. Patients with DLT are followed until toxicities resolve, return to baseline or stabilize.

### Dose Rationale

In preclinical studies, schedules of daily LB-100 dose administration for 3, 4, or 5 days were studied. In the daily x 5 day regimen, rat toxicity data indicated that some renal toxicity was evident following Day 4 administration of LB-100. Thus, a daily x 3 dose of LB-100 is believed to be a safe initial dose and this dosing has been safe in the ongoing Phase 1 clinical trial. Each dose is administered 24 hours apart (+/- 2 hours). Drug is administered in the clinic in order to obtain adequate biomarker assessment. Reported adverse events and potential risks are described in the Experimental Methods section. Appropriate dose modifications for LB 100 are also described in the Experimental Methods section.

LB-100 is supplied as a sterile solution for intravenous administration. Each vial of LB-100 sterile injection contains 10 mL of a 1.0 mg/mL solution of LB-100 in monosodium glutamate, pH 10.5. LB 100 is stored at 20°C (allowable range: 25°C to 10°C). The proper dose is drawn up in a sterile syringe and added to 500 mL of normal saline (0.9%) and is administered intravenously over 2 hours. Dilution in saline reduces the pH so that the infusate is non-irritating, but extravasation is avoided. Following dilution in normal saline, LB-100 is administered within 8 hours.

### General Concomitant Medication and Supportive Care Guidelines

In general, the use of any concomitant medications/therapies deemed necessary for the care of the patient is allowed (unless prohibited, see below). The patient is instructed to notify the investigational site about any new medications she takes after the start of the study drug. The patient is not permitted to take any other anti-cancer therapy while on study (see exception below). Growth factor support is allowed as specified below. All concomitant drugs are reported in the appropriate case report form (CRF) along with dosage information, dates of administration and reasons for use. Patients are asked to record any self-medication; special care should be taken to question patients on any self-medication.

### Prohibited Medications

Erythropoiesis-stimulating agents (ESAs) are not allowed for anemia during the study. G-CSF is allowed during the study for subjects with febrile neutropenia and short term use.

Anticancer therapy (chemotherapy, endocrine, biologic or radiation therapy, and surgery) other than the study treatments must not be given to patients while the patient is enrolled in the treatment portion of the trial. If such agents are required for a patient, then the patient is permanently discontinued from the treatment portion of the study. Exception for breast cancer patients on adjuvant hormonal therapy (e.g. anastrozole/tamoxifen) who have been disease free for at least 2 years.

Other investigational therapies must not be used while the patient is on the study.

Herbal preparations/medications are not allowed throughout the study, as a potential drug-drug interaction is always possible. These herbal medications include: St. John's wort, Kava, ephedra (ma huang), gingko biloba, dehydroepiandrosterone (DHEA), yohimbe, saw palmetto, and ginseng. Patients should stop using these herbal medications at least 7 days prior to first dose of study treatment.

### Duration of Therapy

Subjects are treated for a total of 18 weeks. For subjects responding at week 18, treatment may continue until one of the following criteria applies:
- Dose-limiting toxicity is reached,
- Inter-current illness that prevents further administration of treatment,
- Unacceptable adverse event(s),
- Patient decides to withdraw from the study, or
- General or specific changes in the patient's condition render the patient unacceptable for further treatment in the judgment of the investigator.
- Evidence of disease progression by the IWG 2006 criteria.
- Subjects who wish not to continue treatment will complete their end of study visit at week 18.

### Duration of Follow-Up

Subjects are followed as per calendar on treatment for 18 weeks. After 18 weeks, subjects who continue on study are followed monthly. Off treatment data on AML transformation is updated every 6 months or until death, whichever occurs first. Subjects removed from the study for unacceptable adverse events are followed until resolution or stabilization of the adverse event.

### Criteria for Removal from Study

A subject is considered to have completed the study if the subject meets at least 1 of the following criteria:
- The subject has completed 18 weeks of treatment with study medication with no response.
- The subject died during the study.
- The subject experiences a treatment related AE that led to withdrawal from the study.

A subject may voluntarily withdraw from study medication or withdraw consent from the study at any time. The investigator may also, at his or her discretion, discontinue a subject from participating in the study at any time. The date and the reason for subject withdrawal from the study is recorded.

If the subject is permanently withdrawn from treatment with study medication, but does not withdraw consent, the investigator will make every effort to have the subject complete all withdrawal assessments at the time of withdrawal, and complete all scheduled follow-up visits. Treatment with study medication is discontinued if:
- The subject withdraws consent.
- Further participation would be injurious to the subject's health or wellbeing in the investigator's medical judgment.
- The study is terminated.
- The subject becomes pregnant
- The subjects exhibits leukemic transformation (as evidenced by bone marrow blast counts of at least 20%, or peripheral blast counts of at least 20% lasting at least 8 weeks.
- No clinical benefit has been attained after 16 weeks of treatment.
- Evidence of Disease progression according to IWG 2006 criteria.
- A subject is significantly non-compliant with the requirements of the protocol.
- A subject has an adverse experience that would, in the investigator's judgment, make continued participation in the study an unacceptable risk.

### DOSING DELAYS/MODIFICATIONS

For patients who do not tolerate the protocol-specified dosing schedule, dose adjustments are permitted in order to allow the patient to continue the study treatment. All dose modifications, interruptions or discontinuations are based on the worst preceding toxicity as graded by the NCI Clinical Toxicity Criteria (NCI-CTCAE version 4.03). Once a dose has been reduced during a treatment cycle, re-escalation is not permitted during any subsequent cycle. If the administration of LB-100 is interrupted for reasons other than toxicity, then treatment with the respective study drug may be resumed at the same dose. In general, doses are not reduced for grade 1 toxicities or grade 2 toxicities with resolution on dose interruption (see Table 5), but treatment to control symptoms are provided as appropriate.

If a patient experiences an adverse event that meets DLT criteria (see Example 6), then the patient is taken off study and receive no further treatment. Patients requiring a LB-100 dose delay of >

28 days are permanently discontinued from study drug. Furthermore, patients requiring > 2 dose reductions for LB-100 are permanently discontinued from study drug.

Patients who permanently discontinue all study drugs undergo weekly or every other week follow-up for 30 days after discontinuation of all study treatment or resolution of the AE to ≤ grade 1, whichever occurs first, that includes all study assessments appropriate to monitor the event.

**Table 4: Dose reduction steps for LB-100**

| **DOSE REDUCTION STEPS FOR LB-100** | |
|---|---|
| **LB-100 dose levels and dose reductions*** | |
| Starting dose level | MTD |
| Dose level - 1 | See Table 3 |
| Dose level - 2** | See Table 3 |
| * Dose reduction should be based on the preceding toxicity | |
| ** = If a dose reduction below level - 2 is required, the patient should be discontinued from LB-100 | |

Guidelines for dose modification and dose interruption for toxicities suspected to be related to LB-100 are described in Table 5. Treatment is resumed at a lower dose:
- If the same toxicity recurs with the same severity, then the next treatment re-initiation must resume at a lower dose irrespective of duration.
- If the same toxicity recurs with a worse severity, then the patient must discontinue treatment with LB-100.

**Table 5:**

| **LB-100 - Recommended dose modifications and criteria for treatment interruption and re-initiation with treatment-related adverse events** | |
|---|---|
| **Worst toxicity (CTCAE 4.03 Grade)**** | **Dose Modifications for LB-100** |
| **HEMATOLOGICAL** | |
| **Neutropenia (ANC)** | |
| Severe Febrile neutropenia (ANC < 0.5 x 10⁹/L, temperature of ≥ 38°C and ICU admission) | Omit dose until resolved, then↓ 1 dose level |

| Thrombocytopenia | |
|---|---|
| Grade 4 (PLT < 25 x 10⁹/L) AND major bleeding event | Omit dose until bleed resolved, then↓ 1 dose level |

| **RENAL** | |
|---|---|
| **Serum creatinine** | |
| Grade 1 (< 2 x ULN) | Maintain dose level |
| Grade 2 (2-3 x ULN) | Omit dose until resolved to ≤ grade 1, then: |
| | If resolved in ≤ 7 days, then maintain dose level |
| | If resolved in > 7 days, then↓ 1 dose level |
| Grade 3 (> 3.0 - 6.0 x ULN) | Permanently discontinue patient from LB-100 |
| Grade 4 (> 6.0 x ULN) | Permanently discontinue patient from LB-100 |

| **Hematuria** | |
|---|---|
| Grade 1 (asymptomatic) | Maintain dose level |
| Grade 2 (symptomatic) | Omit dose until resolved to ≤ grade 1, then: |
| | If resolved in ≤ 7 days, then maintain dose level |
| | If resolved in > 7 days, then↓ 1 dose level |
| Grade 3 | Permanently discontinue patient from LB-100 |
| Grade 4 | Permanently discontinue patient from LB-100 |

| **CARDIAC** | |
|---|---|
| Symptomatic, response to intervention, ejection fraction 20-39% or > 20% drop from baseline | • Omit LB-100 until resolved* (as defined below), then ↓ 1 dose level |
| | • LVEG measurement to be repeated, if not resolved* within 21 days, permanently discontinue patient from LB-100 treatment |
| Refractory or poorly controlled, ejection fraction < 20% | Permanently discontinue patient from LB-100 |
| Grade 4 (> 10.0 x ULN) | Permanently discontinue patient from LB-100 |
| * the event is considered resolved when the patient is asymptomatic, has a resting ejection fraction ≥ 40% and ≤ 20% decrease from baseline | |

| **AST or ALT** | |
|---|---|
| Grade 1 (> ULN - 3.0 x ULN) | Maintain dose level with LFTs monitored per protocol |
| Grade 2 (> 3.0 - 5.0 x ULN) without total bilirubin elevation to > 2.0 x ULN | Omit dose until resolved to ≤ grade 1, then: |
| | If resolved in ≤ 7 days, then maintain dose level |
| | If resolved in > 7 days, then↓ 1 dose level |
| Grade 3 (> 5.0 - 20.0 x ULN) without total bilirubin elevation to > 2.0 x ULN | Permanently discontinue patient from LB-100 |
| Grade 4 (> 20.0 x ULN) without bilirubin elevation to > 2.0 x ULN | Permanently discontinue patient from LB-100 |

| **AST or ALT and concurrent Bilirubin** | |
|---|---|
| AST or ALT > 3.0 x ULN and total bilirubin > 2.0 x ULN | Permanently discontinue patient from LB-100 |
| ** Common Technology Criteria for Adverse Events (CTCAE) version 4.03. | |

### Definition of an AE

Any untoward medical occurrence in a subject or clinical investigation subject, temporally associated with the use of a medicinal product, whether or not considered related to the medicinal product. Note: An AE can therefore be any unfavorable and unintended sign (including an abnormal laboratory finding), symptom, or disease (new or exacerbated) temporally associated with the use of a medicinal product.

Events meeting the definition of an AE include:
- Exacerbation of a chronic or intermittent pre-existing condition including either an increase in frequency and/or intensity of the condition.
- New conditions detected or diagnosed after investigational product administration even though it may have been present prior to the start of the study.
- Signs, symptoms, or the clinical sequelae of a suspected interaction
- Signs, symptoms, or the clinical sequelae of a suspected overdose of either investigational product or a concomitant medication (overdose per se will not be reported as an AE/SAE). "Lack of efficacy" or "failure of expected pharmacological action" per se within the duration of initial LB-100 treatment/exposure of 18 weeks are not reported as an AE or SAE. However, the signs and symptoms and/or clinical sequelae resulting from lack of efficacy are reported if they fulfill the definition of an AE or SAE.

Events that do not meet the definition of an AE include:
- Medical or surgical procedure (e.g., endoscopy, appendectomy); the condition that leads to the procedure is an AE.
- Situations where an untoward medical occurrence did not occur (social and/or convenience admission to a hospital).
- Anticipated day-to-day fluctuations of pre-existing disease(s) or condition(s) present or detected at the start of the study that do not worsen.
- The disease/disorder being studied or expected progression, signs, or symptoms of the disease/disorder being studied, unless more severe than expected for the subject's condition.
- Death due to the disease being studied.

### Definition of an SAE

A serious adverse event is any untoward medical occurrence that, at any dose:
- Results in death
- Is life-threatening NOTE: The term "life-threatening" the definition refers to an event in which the subject was at risk of death at the time of the event. It does not refer to an event, which hypothetically might have caused death, if it were more severe.
- Requires hospitalization or prolongation of existing hospitalization. NOTE: In general, hospitalization signifies that the subject has been detained (usually involving at least an overnight stay) at the hospital or emergency ward for observation and/or treatment that would not have been appropriate in the physician's office or out-patient setting. Complications that occur during hospitalization are AEs. If a complication prolongs hospitalization or fulfills any other serious criteria, the event is serious. When in doubt as to whether "hospitalization" occurred or was necessary, the AE should be considered serious. Hospitalization for elective treatment of a pre-existing condition that did not worsen from baseline is not considered an AE.

- Results in disability/incapacity NOTE: The term disability means a substantial disruption of a person's ability to conduct normal life functions. This definition is not intended to include experiences of relatively minor medical significance such as uncomplicated headache, nausea, vomiting, diarrhea, influenza, and accidental trauma (e.g. sprained ankle) which may interfere or prevent everyday life functions but do not constitute a substantial disruption.
- Is a congenital anomaly/birth defect
- All treatment related grade 4 non-hematologic laboratory abnormalities assessed using the NCI CTCAE v 4.03.

Medical or scientific judgment is exercised in deciding whether reporting is appropriate in other situations, such as important medical events that may not be immediately life-threatening or result in death or hospitalization but may jeopardize the subject or may require medical or surgical intervention to prevent one of the other outcomes listed in the above definition. These are also considered serious.

Examples of such events are invasive or malignant cancers, intensive treatment in an emergency room or at home for allergic bronchospasm, or convulsions that do not result in hospitalization, or development of drug dependency or drug abuse.

### Relationship to Investigational Product

It is a regulatory requirement for investigators to assess relationship to investigational product based on information available. The assessment is reviewed on receipt of any new information and amended if necessary. "A reasonable possibility" is meant to convey that there are facts/evidence or arguments to suggest a causal relationship. Facts/evidence or arguments that may support "a reasonable possibility" include, e.g., a temporal relationship, a pharmacologically-predicted event, or positive dechallenge or rechallenge. Confounding factors, such as concomitant medication, a concurrent illness, or relevant medical history, are also considered.

### Laboratory and Other Safety Assessment Abnormalities Reported as AEs and SAEs

Any abnormal laboratory test results (hematology, clinical chemistry, or urinalysis) or other safety assessments (e.g., ECGs, radiological scans, vital signs measurements), including those that worsen from baseline are recorded as per the NCI-CTCAE criteria. However, these laboratory results are recorded as AEs or SAEs if deemed clinically significant in the medical and scientific judgment of the investigator or treating physician. Any clinically significant safety assessments that are associated with the underlying disease are not reported as AEs or SAEs, except for findings judged by the investigator or treating physician to be more severe than expected for the subject's condition or death. Data is collected for typical disease-related events such as anemia, leukopenia or worsening of thrombocytopenia.

All infections experienced during the study are recorded as AEs or SAEs.

### Disease-Related Events and/or Disease-Related Outcomes Not Qualifying as SAEs

The following conditions do not qualify as an AE or SAE provided they are not considered attributable to study medication:
- events that occur prior to the 1st dose of LB-100.
- cases of disease progression.

### Pregnancy

Any pregnancy that occurs during study participation is reported. To ensure subject safety, each pregnancy is reported to the FDA with carbon copy (CC) notification to LB-100 within 2 weeks of learning of its occurrence. The pregnancy is followed up to determine outcome (including premature termination) and status of mother and child. Pregnancy complications and elective terminations for medical reasons are reported as an AE or SAE. Spontaneous abortions are reported as an SAE. Any SAE occurring in association with a pregnancy, brought to the investigator's attention after the subject has completed the study and considered by the investigator as possibly related to the investigational product, is promptly reported to the pharmacovigiliance group. In addition, pregnancy information on any female partners of male study subjects who become pregnant while the subject is enrolled in the study is collected. Pregnancy information is reported as described above.

### Time Period and Frequency of Detecting AEs and SAEs

The investigator or site staff is responsible for detecting, documenting and reporting events that meet the definition of an AE or SAE. AEs are collected from the start of Investigational Product and through the follow-up contact. SAEs are collected over the same time period as stated above for AEs. However, any SAEs assessed as related to study participation (e.g., investigational product, protocol mandated procedures, invasive tests, or change in existing therapy) are recorded from the time a subject consents to participate in the study up to and including any follow-up contact. All SAEs are reported, as indicated.

### Prompt Reporting of Serious Adverse Events and Other Events to the FDA with notification to Lixte Biotechnology

Any serious adverse events which occur during the clinical study or within 30 days of receiving the last dose of study medication, whether or not related to the study drug, are reported by the investigator. In addition, any SAEs which occur as a result of protocol specific diagnostic procedures or interventions are also reported. SAEs brought to the attention of the investigator at any time after cessation of LB-100 and considered by the investigator to be related or possibly related to LB-100 are reported to the FDA if and when they occur.

Additionally, in order to fulfill international reporting obligations, SAEs that are related to study participation (e.g., procedures, invasive tests, change from existing therapy) or are related to a concurrent medication are collected and recorded from the time the subject consents to participate in the study until he/she is discharged.

**Table 6: Reporting of SAEs**

| | **Initial Reports** | | **Follow-up Information on a Previous Report** | |
|---|---|---|---|---|
| **Type of Event** | **Time Frame** | **Documents** | **Time Frame** | **Documents** |
| All SAEs | 24 hours* | "SAE" data collection tool | 24 hours* | Updated "SAE" data collection tool |
| Pregnancy | 2 Weeks* | Pregnancy Notification Form | 2 Weeks* | Pregnancy Follow-up Form |
| *From the time point when the SAE or pregnancy became known to reporter | | | | |

### Regulatory Reporting Requirements for SAEs

Prompt notification of SAEs by the investigator to the FDA (and Lixte Biotechnology) is essential so that legal obligations and ethical responsibilities towards the safety of subjects are met. The sponsor-investigator has a legal responsibility to notify both the local regulatory authority and other regulatory agencies about the safety of a product under clinical investigation. The sponsor-investigator will comply with specific regulatory requirements relating to safety reporting to the regulatory authority, Institutional Review Board (IRB), the FDA, notification to Lixte Biotechnology, and sub-investigators. Investigator safety reports are prepared for suspected unexpected serious adverse reactions according to local regulatory requirements and those policies set forth by the FDA and are forwarded to investigators and Lixte Biotechnology as necessary. An investigator who receives an investigator safety report describing an SAE(s) or other specific safety information (e.g., summary or listing of SAEs) from the H. Lee Moffitt Cancer Center will file it with the CIB and will notify the IEC /IRB, if appropriate according to local requirements.

**Table 7: International Prognostic Scoring System (IPSS) for Myelodysplatic Syndromes**

| **Characteristic** | **Score** | | | | |
|---|---|---|---|---|---|
| | **0** | **0.5** | **1.0** | **1.5** | **2.0** |
| Bone marrow blasts (%) | <5 | 5-10 | - | 11-20 | 21-30 |
| Karyotype* | Good | Intermediate | Poor | | |
| Number of Cytopenias | 0-1 | 2-3 | | | |

| | | | | | |
|---|---|---|---|---|---|
| *Good = normal, -y, del(5q), or del(20q); Poor = chromosome 7 abnormalities or complex (≥3) abnormalities; Intermediate = all others Total score: 0 = low risk, 0.5-1.0 = intermediate-1, 1.5-2.0 = intermediate-2, >2.0 = high | | | | | |

**Table 8: WHO Classification for MDS**

| **WHO Category** | **Peripheral Blood** | **Bone Marrow** |
|---|---|---|
| Refractory cytopenias with unilineage dysplasia (RCUD): (refractory anemia (RA), refractory neutropenia (RN), refractory thrombocytopenia (RT)) | Unicytopenia | Unilineage dysplasia (>10% in one myeloid lineage) |
| | <1% blasts | |
| | < 1x10FF9FF monocytes | < 10 % myeloid or megakaryocytic dysplasia |
| | | < 5% blasts |
| | | < 15% sideroblasts |
| Refractory anemia with ring | Anemia | Erythroid dysplasia |
| | < 1% blasts | < 10 % myeloid or megakaryocytic dysplasia |
| sideroblasts (RARS) | < 1x10FF9FF monocytes | |
| | | < 5% blasts |
| | | > 15% sideroblasts |
| Refractory cytopenia with | Bi-or pancytopenia | Dysplasia in > 10% of the cells in 2 or more cell lines |
| multilineage dysplasia (RCMD) | < 1%blasts | < 5% blasts |
| | <1x10FF9FF monocytes | +/- 15% sideroblasts (RCMD-RS) |
| Refractory anemia with multilineage dysplasia | Bi-or pancytopenia | Dysplasia in > 10% of the cells in 2 or more cell lines |
| and | < 1%blasts | < 5% blasts |
| ring sideroblasts (RCMD-RS) | <1x10FF9FF monocytes | > 15% sideroblasts |
| Refractory anemia with excess | Cytopenia | Uni or multilineage dysplasia |
| | Type I: 1-5% blasts | Type I 5-9% blasts |
| blasts type I & II (RAEB-1 & RAEB II) | | Type II 10-19% blasts |
| | Type II: 5-19% blasts | |
| MDS associated with isolated | Anemia Normal or elevated platelets < 5% blasts | Normal or increased megakaryocytes |
| | | < 5% blasts |
| del (5q) | | |
| MDS unclassified (MDS-U) | Cytopenia | unilineage dyplasia of myeloid or megakaryocytic line |
| | <1% blasts | |
| | | < 5% blasts |

### Pharmaceutical Information

LB-100 is supplied as a sterile solution for intravenous administration. LB-100 is to be stored at -20°C (allowable range: -10°C to -25°C (or lower)). Each vial contains LB-100 at a concentration of 1 mg/ml.

### Study drug compliance and accountability

Compliance is assessed by the investigator and/or study personnel at each patient visit using pill counts and information provided by the patient and/or caregiver. Records of study medication used, dosages administered, and intervals between visits and the completion of the study are captured in the Drug Accountability Form. This information is captured in the source document at each patient visit.

### Study drug accountability

The investigator or designee maintains an accurate record of the shipment and dispensing of study treatment in a drug accountability ledger. Drug accountability is noted by the field monitor during site visits and at the completion of the study.

At study close-out, and, as appropriate during the course of the study, the investigator returns all unused study treatment, packaging, drug labels, and a copy of the completed drug accountability ledger to Lixte Biotechnology.

### Disposal and destruction

The drug supply can be destroyed at Drug Supply group or third party, as appropriate. Study drug destruction at the investigational site will only be permitted if authorized by Lixte Biotechnology in a prior agreement and if permitted by local regulations.

### Study Calendar

All screening evaluations are performed within 4 weeks prior to the start of LB-100 treatment. Subjects have a bone marrow biopsy and aspirate (including cytogenetics) performed within 4 weeks prior to the start of treatment. All transfusion and pre-transfusion hemoglobin or platelet counts are recorded for the 8 weeks prior to initiation of study treatment. Strict adherence to the visit schedule is required. In the event that a visit or test cannot be scheduled on the exact visit day, a window of ± 7 days is allowed for scheduling. Bone marrow aspiration and biopsy exams are done within a 28 day window of the allotted date. Tests (including bone marrow biopsies and aspirates) done within the screening period prior to signing informed consent are allowed for use in this study.

### Baseline Assessment

### Is done within 4 weeks of starting treatment.

Medical history including:
- Disease characteristics such as WHO subtype, IPSS score, prior treatments
- ECOG performance status (see Table 1)
- Concurrent medication review
- Routine physical examination
- Bone marrow examination, including cytomorphology, cytogenetic assessment, flow cytometry analysis, and molecular analysis with next-generation sequencing (NGS) myeloid panel

### Laboratory assessments:

- CBC with differential
- Clinical chemistries including BUN/urea, creatinine, sodium, potassium, alkaline phosphatase, alanine aminotransferase (ALT), aspartate aminotransferase (AST), total bilirubin and albumin
- Urine analysis
- Urine or serum pregnancy test for females of childbearing potential will be performed at Day -1 or on Day 1, prior to first dose of study medication.
- Review and record any blood and blood supportive care products for the prior 8 weeks.

**Table 9: ECOG Performance Status Criteria**

| **ECOG Performance Status Scale** | |
|---|---|
| **Grade** | **Descriptions** |
| **0** | Normal activity. Fully active, able to carry on all pre-disease performance without restriction |
| **1** | Symptoms, but ambulatory. Restricted in physically strenuous activity, but ambulatory and able to carry out work of a light or sedentary nature (e.g., light housework, office work). |
| **2** | In bed <50% of the time. Ambulatory and capable of all self-care, but unable to carry out any work activities. Up and about more than 50% of waking hours. |
| **3** | In bed >50% of the time. Capable of only limited self-care, confined to bed or chair more than 50% of waking hours. |
| **4** | 100% bedridden. Completely disabled. Cannot carry on any self-care. Totally confined to bed or chair. |
| **5** | Dead. |

### Treatment Timeline

Treatment Period (weeks 1-18): LB-100 is administered intravenously on days 1-3 for a 3 week treatment cycle. Subjects undergo a CBC with leukocyte differential and a complete metabolic profile (CMP) weekly or more frequently per the study investigator. A BM aspirate and biopsy with cytogenetic analysis and NGS myeloid panel is performed after cycle 3 and 6 (weeks 9, 18) to assess pathologic response, cytogenetic response, molecular response and disease progression.

Week 18 End of Treatment: Subjects complete a response assessment. Subjects discontinuing study early complete their end of treatment visit within two weeks after their last dose of investigational product. Physical exam, vital signs, concomitant medication, adverse event reporting, CBC, CMP and BM aspirate and biopsy with cytogenetic analysis and NGS myeloid panel are performed.

Continuation Phase: After completing cycle 6 response assessments, some responders continue to receive LB-100. Bone marrow biopsy and aspirate are repeated after every 6 cycles. A CBC and CMP is obtained monthly or more frequently per study investigator and complete metabolic profile as per standard of care.

Off treatment assessment: includes best response achieved, date of first response, date of loss of response, reason for discontinuation.

Off treatment evaluation: include vital status, date of death/last contact, transformation to AML and the date of transformation to AML if applicable. This evaluation is updated every 6 months for 2 years.

| **Table 10: Study Calendar** | | | | | | | | | | | | | | |
|---|---|---|---|---|---|---|---|---|---|---|---|---|---|---|
| **Study Calendar** | **Pretreat ment** | **Cycle 1** | | | | | | **Cycle 2 and Subsequent Cycles** | | | | | **After every 3 cycles** | **Off-Study** ** |
| **Evaluation** | | Day 1 | Day 2 | Day 3 | Day 8^{a} | Day 15^{a} | Day 22^{b} | Prior to/ Day 1^{b} | Days 2 & 3 | Day 8^{a} | Day 15^{a} | Day 22^{b} | | |
| Informed consent | X | | | | | | | | | | | | | |
| Medical history | X | | | | | | | | | | | | | |
| Physical exam | X | X | | | | | | X | | | | | | X |
| Height | X | | | | | | | | | | | | | |
| Weight | X | X | | | | | | X | | | | | | X |
| Vital signs^{c} | X | X | X | X | | | X | X | X | | | X | | X |
| ECOG PS | X | X | | | | | | X | | | | | | X |
| Bone marrow Biopsy and Aspiration | X | | | | | | | | | | | | X | X |
| Flow Cytometry and Cytogenetics | X | | | | | | | | | | | | X | X |
| NGS Myeloid Panel | X | | | | | | | | | | | | X | X |
| Correlative Studies | X | | | | | | | | | | | | X | |
| Response Assessment | | | | | | | | | | | | | X | X |
| Hematology^{d} | X | X | | X | X | X | X | X | | X | X | X | | X |
| Blood Chemistry^{e} | X | X | | X | X | X | X | X | | X | X | X | | X |
| Urinalysis | X | X | | X | X | X | X | X | | | | X | | X |
| Pregnancy test | X^{T} | | | | | | | | | | | | | |

| | | | | | | | | | | | | | | |
|---|---|---|---|---|---|---|---|---|---|---|---|---|---|---|
| PK blood sampling^{g} | | X | | X | | | | | | | | | | |
| PD blood sampling | | X | | X | | | | | | | | | | |
| Transfusion Log | X | | | | | | | | | | | | | X |
| Best Response | | | | | | | | | | | | | | X |
| Concomitant medications | X | < --------------------------------------------- throughout study ---------------------------------------------> | | | | | | | | | | | | X |
| Adverse events | | <--------------------------------------------- throughout study ---------------------------------------------> | | | | | | | | | | | | X |
| LB-100 is administered IV on days 1-3 of each 21 day cycle. | | | | | | | | | | | | | | |
| a: in case of scheduling conflicts on the specified Days 8 and 15, ± 3 day windows can apply. | | | | | | | | | | | | | | |
| b: "Day 22" = Day 1 of next cycle for patients continuing treatment. Day 1 evaluations for subsequent cycles are done within 3 days prior to next cycle drug administration. These tests are not repeated if done on Day 22 of prior cycle. | | | | | | | | | | | | | | |
| c: vital signs including blood pressure, heart rate, respiration rate, and temperature. Part 1, on Days 1-3: before LB-100 infusion, within 15 minutes after end of infusion, and at 2 hours after end of infusion. | | | | | | | | | | | | | | |
| d: hematology including hemoglobin, WBC with differential, and platelet count. | | | | | | | | | | | | | | |
| e: blood chemistry including sodium, potassium, BUN, glucose, SGOT/SGPT (ALT/AST), alkaline phosphatase, total protein, total bilirubin, albumin, creatinine, and calcium. | | | | | | | | | | | | | | |
| f: pregnancy test; for women of childbearing potential, a negative pregnancy test (urine or serum) is documented at Day -1 or Day 1 prior to 1st dose of medication | | | | | | | | | | | | | | |
| g: phase 1 portion of the trial only | | | | | | | | | | | | | | |
| A11 screening/pre-treatment evaluations are performed within 4 weeks prior to the start of LB-100 treatment. | | | | | | | | | | | | | | |
| Week 18 Evaluation and End of Treatment: Subjects complete a response assessment within one week after their last administration | | | | | | | | | | | | | | |
| of LB-100. Subjects discontinuing study early complete their end of treatment visit within two weeks after their last dose of investigational product. Physical exam, vital signs, concomitant medication, adverse event reporting, CBC, and blood chemistry and BM aspirate and biopsy with cytogenetic analysis will be performed. | | | | | | | | | | | | | | |
| **Continuation Phase: After completing cycle 6 response assessments, some responders (HI and/or cytogenetic response) continue | | | | | | | | | | | | | | |
| to receive LB-100 on 21 day cycles. Bone marrow biopsy and aspirate are repeated after every 6 cycles. A CBC and CMP is obtained every 4 weeks or more often per study investigator. The off treatment assessment is done within a week off treatment. | | | | | | | | | | | | | | |
| Off Treatment assessment: includes best response, date of first response, date of loss of response, reason for discontinuation. | | | | | | | | | | | | | | |
| Off study follow up: include vital status, date of death/last contact, transformation to AML and the date of transformation to AML if applicable. This evaluation is updated every 6 months for 2 years. | | | | | | | | | | | | | | |
| **** all dates are +/- one week | | | | | | | | | | | | | | |

### MEASUREMENT OF EFFECT

### Definitions:

Response and progression is assessed according to modified International Working Group (IWG) 2006 criteria (Tables 1 and 2) (Cheson, B.D. et al., 2006). Improvements must last ≥ 8 weeks.

Erythroid Response for pretreatment hemoglobin < 11 g/dl:
- ≥ 1.5 g/dL increase in hemoglobin.
- For transfused subjects having pre-transfusion baseline hemoglobin ≤ 9 g/dL, a reduction of 4 or more RBC units in the previous 8 weeks compared with pretreatment transfusion number in the previous 8 weeks.

Platelet response for subjects with a pre-treatment platelet count < 50 x 10⁹/L:
- Major platelet response: An absolute increase of ≥ 30 x 10⁹/L. If platelets are < 20 x 10⁹/L at baseline, then a 100% increase will qualify as a major platelet response. If subjects are transfusion dependent at baseline, platelet transfusion independence sustained for 8 consecutive weeks will qualify as a major platelet response.
- Complete platelet response: increase of platelet count to ≥ 100 x 10⁹/L for 8 consecutive weeks.

Neutrophil response with pretreatment ANC < 1 x 10⁹/L:
- ≥ 100% increase and an absolute increase of > 0.5 x 10⁹/L

Progression/relapse following hematological improvement: At least one of the following:
- Any newly developed (RBC/platelet) transfusion dependence,
- ≥ 50% decrease from maximum response levels in granulocytes or platelets,
- or Reduction of ≥ 1.5 g/dL hemoglobin.

### Complete Response (CR)

- Bone marrow: ≤ 5% myeloblasts with normal maturation of all cell lines
- Persistent dysplasia is noted
- Peripheral blood:
   ∘ Hemoglobin ≥ 11 g/dL
   ∘ Platelets ≥ 100 x 10⁹/L
   ∘ Neutrophils ≥ 1.0 x 10⁹/L
   ∘ BLASTS 0%

### Partial Response (PR)

- All CR criteria if abnormal before treatment, except:
- Bone marrow blasts decreased by ≥ 50% over pretreatment but still > 5%
- **Cellularity and morphology not relevant**

### Marrow Complete Response (mCR)

- Bone marrow: ≤ 5% myeloblasts and decrease by ≥ 50% over pretreatment
- **Peripheral blood: if HI responses, they are noted in addition to marrow CR**

### Stable Disease (SD)

- Failure to achieve at least PR, but no evidence of progression for > 8 weeks

### Duration of Response:

The duration of response is defined as the time between achieving the primary endpoint until the first date that disease progression defined by the bone marrow response outlined above, progression/relapse following a CR, marrow CR or PR, or progressions/relapse following hematological improvement (HI) as outlined above.

Pathologic Response: Pathologic response is categorized as a PR, CR, or mCR. Response parameters in the peripheral blood and/or bone marrow must be sustained for at least 4 weeks. See Tables 1 and 2.

### STATISTICAL CONSIDERATIONS

### Study Design

Clinical trials are conducted as single institution, open-label, Phase 1b/2 clinical trials evaluating the toxicity and efficacy of intravenous LB-100 in lower risk MDS patients that are conducted in 2 parts: a Phase 1 Dose Finding part followed by a Simon's two-stage Phase 2 design. A stopping rule is used to monitor for too many unacceptable adverse events, and patients are monitored for the transformation to acute myeloid leukemia as defined by WHO (see Tables 1 and 2). Eligible subjects have a WHO diagnosis of MDS or MDS/MPN and meet IPSS criteria for low or int-1 risk disease and have previously failed a hypomethylating agent or lenalidomide (lenalidomide failure/intolerance mandatory for del(5q) patients).

In the Finding Phase, patients receive intravenous infusions of LB-100 over 15 minutes on days 1-3 of each 21 day cycle at escalating doses starting at Dose Level 1 (see Table 3). Patients are followed for at least 6 weeks (2 cycles) before the safety of each cohort can be fully assessed and decisions made for dose escalation in the next cohort. The MTD is defined as the dose level below which DLT is manifested in ≥33% of the patients or at dose level 2 if DLT is manifested in <33% of the patient. Following completion of the Dose Finding Phase, patients undergo a dose expansion, whereby patients will be treated with LB-100 administered intravenously at the MTD using the 21 day cycle as determined from the Dose Escalation Phase. Within the cohort of patients treated at the MTD, a stopping rule is used to monitor for too many unacceptable adverse events.

### Sample Size/Accrual Rate

The number of patients enrolled depends on the number of dose levels evaluated before reaching DLT. Three to 6 evaluable patients are entered per dose level. Up to 12 evaluable patients are treated in the Phase 1 portion of the study. Up to 35 additional evaluable patients are treated at the level of the MTD in the Phase 2 portion of the trial, which provides sufficient power to detect an improvement in the response rate, as detailed in the Experimental Methods section. The maximum total accrual is 47 patients.

### Statistical Analysis Methods

Demographic and clinical variables for the study patients are summarized using descriptive statistics (mean, standard deviation, median, inter-quartile range, range, frequency counts and percentages). Safety and efficacy data are analyzed overall as well as separately for each dose cohort when appropriate.

Within the Phase 2 portion for the cohort of patients treated at the MTD, a Simon's two-stage design is applied, as follows:
- Enroll a total of 21 evaluable patients at the MTD (including patients who were enrolled during the Phase 1 part of the study). Where there are 2 or fewer responders, as defined by the IWG 2006 criteria (HI and/or cytogenetic response), the study is terminated early with the conclusion that the regimen does not warrant further investigation. Where there are 3 or more responders, enrollment is continued to Stage 2.
- Stage 2: Enroll 20 more evaluable patients for a total of 41. Where there are 6 or fewer responders, there is insufficient evidence to continue, and study of this treatment is discontinued. Where there are 7 or more responders, there is sufficient evidence, and further study of LB-100 is continued in Phase 3.

This rule has the following operating characteristics: 90% power, with alpha=0.1 under the null hypothesis that the response rate is <10% versus that alternative hypothesis that it is ≥26%. There is an expected sample size of 28.03, and a probability of early termination of 0.648.

### Safety Analysis

This analysis includes all subjects who have received any protocol treatment, regardless of patient eligibility. The number (%) of subjects with adverse events, serious adverse events, and adverse events leading to treatment discontinuation is reported. Adverse events summaries are reported by type and severity.

Laboratory parameters are also summarized using descriptive statistics. The following Simon's two-stage design is used to monitor for the occurrence of too many unacceptable adverse events, defined as non-hematological grade 3/4 toxicity.
Stage 1: Enroll 21 evaluable patients. If at any time, 5 or more patients have a non-hematological grade ≥3 adverse event, then the study is temporarily halted for consideration of dose modifications or closure. If less than 5 patients have a non-hematological grade ≥3 adverse events, the study continues to Stage 2.
Stage 2: Enroll 20 more evaluable patients, for a total of 41. If at any time, 12 or more patients have a non-hematological grade ≥3 adverse event, then the study is temporarily halted for consideration of dose modifications or considered not sufficiently safe. If less than 12 patients have a non-hematological grade ≥3 adverse events, then the therapy is considered sufficiently safe.

In addition, the patients are monitored for the transformation to acute myeloid leukemia as defined by WHO (see Tables 1 and 2). If at any time, 2 evaluable patients transform to acute myeloid leukemia, then the study is temporarily halted for review, and there is consideration of dose modifications or closure.

### Efficacy Analysis: Intention to Treat (ITT)

This analysis includes all subjects who have received any protocol treatment, regardless of patient eligibility or duration of treatment. Those who have no response assessment data due to reasons such as drop out of the study, withdrawal consent, or lost to follow-up are treated as non-responders for various response evaluations. The proportion of subjects achieving the primary endpoint is summarized. A 95% exact binomial confidence interval of the proportion is provided for all participants treated at the MTD. In addition, a second analysis of evaluable subjects is performed. Evaluable subjects are defined as those who complete at least 9 weeks of therapy and complete the first on treatment bone marrow biopsy and aspirate to evaluate study drug response.

### Analyses of Study Endpoints

To address the primary objective for Phase 1b, the 3+3 design above is applied.

To address the primary objective for Phase 2, the Simon's two-stage design above is applied.

Secondary objective 3 - Within the cohort of patients on the Phase 1 portion of the study: For plasma concentrations, calculations of the maximum concentration, time to reach maximum concentration, area under the curve, half-life, total body clearance, and volume of distribution) are determined. Descriptive statistics, including means, standard deviations, confidence intervals, medians, quartiles, minimums, and maximums, are presented for these measures.

Secondary objective 4 - Within patients who have del(5q) MDS, the proportion of patients who achieve HI and/or cytogenetic response is calculated. 95% confidence intervals are placed on these proportions.

Secondary objective 5 - Within the patients on the Phase 2 portion of the study who have been treated at the MTD, the duration of response is calculated. Duration of response is defined as the time from achievement of HI and/or cytogenetic response until relapse or progression of disease, or death due to disease. The mean and standard deviation of the duration is calculated, and a 95% confidence interval is placed on this mean.

Secondary objective 6 - The incidence rate for transformation to Acute myeloid leukemia (AML) is calculated according to World Health Organization (WHO) criteria. A 95% confidence interval is placed on these proportions.

Secondary objective 7 -PP2A activity via Active PP2A assay kit is calculated. This measure (continuous value) is collected pre- and post-protocol therapy. A Wilcoxon signed rank test is used to test for statistically significant changes from baseline to post-therapy. The mean, standard deviation, median, quartiles, minimum, and maximum expression of PP2A substrates (i.e. CDC25C, MDM2, AKT) and p53 from bone marrow (BM) samples are calculated.

Secondary objective 8 - The mean, standard deviation, median, quartiles, minimum, and maximum erythropoietin-induced STAT5 activation in erythroid progenitors is calculated.

Secondary objective 9 - Recurrent gene mutations in *ABL1, ASXL1, CBL, CEBPA, CSF3R, CUX1, DNMT3A, ETV6, EZH2, FLT3, IDH1, IDH2, IKZF1, JAK2, KIT, KRAS, MLL, MPL, MYD88, NPM1, NRAS, PHF6, RUNX1, SETBP1, SF3B1,* SH2B3, *SRSF2, TET2, TP53, U2AF1, WT1,* and *ZRSR2* are determined at study entry and at best response/end of study and/or progression of disease. This analysis is exploratory in nature.

Preliminary data on the above endpoints is very useful for further investigation of this protocol's treatment. Such data is summarized appropriately in an exploratory fashion. Both point estimates and 95% confidence intervals are reported, if feasible.

### Pharmacokinetics

Plasma sampling for pharmacokinetic (PK) measurements of LB-100 are performed in Phase 1 only. Blood samples are collected on Cycle 1 Days 1 and 3 as per the schedule in Table 11. At each timepoint, 5 mL is drawn into chilled heparin collection tubes. Collection tubes are kept on ice until the plasma is separated.

The collection tubes are centrifuged (1800 rcf for 10 minutes) in a refrigerated centrifuge at approximately 4°C within 30 minutes of collection. Plasma is aliquoted (two aliquots) into appropriately labeled polypropylene tubes (1.8-2 mL cryovials) containing 0.5N NaOH. For every 1.0 mL of plasma aliquoted 0.1 mL of 0.5N NaOH is added. Samples are stored at -70° C until the time of analysis.

**Table 11: Pharmacokinetic Sampling for Part 1**

| **Study Day** | **Draw Time** | **One (1) 5 mL Heparin for LB-100** |
|---|---|---|
| Day 1 | pre-dose | X |
| | immediately at end of LB-100 infusion | X |
| | 15 minutes (±2 minutes) post LB-100 infusion | X |
| | 30 minutes (± 2 minutes) post LB-100 infusion | X |
| | 1 hour (± 5 minutes) post LB-100 infusion | X |
| | 2 hours (± 5 minutes) post LB-100 infusion | X |
| | 4 hours (± 5 minutes) post LB-100 infusion | X |
| Day 3 | pre-dose | X |
| | immediately at end of LB-100 infusion | X |
| | 15 minutes (± 2 minutes) post LB-100 infusion | X |
| | 30 minutes (± 2 minutes) post LB-100 infusion | X |
| | 1 hour (± 5 minutes) post LB-100 infusion | X |
| | 2 hours (± 5 minutes) post LB-100 infusion | X |
| | 4 hours (± 5 minutes) post LB-100 infusion | X |

### Sample Collection

At the time of the first dose and third dose of cycle 1 and day 1 of subsequent cycles, 4 green tops and one red top vial are collected. Study personnel collect the sample from the laboratory draw area (within 3 hours of blood draw) . The green top samples (heparinized) are processed using RBC lysis buffer to remove RBC and debris. They are additionally processed by centrifugation (1700rpm for 20 minutes) with FICOLL to collect the mononuclear cellular layer.

This layer is cryopreserved as previously described and stored in liquid nitrogen for later use labeled with a unique identifier that corresponds to each patient known only to the investigator and study personnel. The red top samples are processed by centrifugation and the supernatant (serum) collected and cryopreserved for later use labeled with a unique identifier that corresponds to each patient known only to the investigator and study personnel.

### PP2A activity as a pharmacodynamic marker for LB-100.

At the time of the first dose and on day 3 peripheral blood is collected from the subjects (may be up to one hour prior to first dose), processed and stored as above. Peripheral blood is also drawn three hours after the first dose (+/- 30 minutes) and is collected, processed and stored as above.

Peripheral blood is also collected at the assigned time points (see study calendar) and processed as above. These samples are batched for analysis after 10 samples have been processed and cryopreserved. They are reconstituted in STEM span and 20% FBS in the laboratory of Alan List. PP2A activity is measured by Active PP2A assay kit as previously described utilizing pre and post LB-100 dose as described above.

### Bone Marrow Aspirate and biopsy analyses

Using portion of cryopreserved bone marrow aspirates, western blot analysis of total and phosphorylated forms of MDM2, Cdc25C, AKT, p53, cereblon and CSNK1A1 are measured.

Immunohistochemistry of p53 is performed and is reported as 0-3+. Erythropoietin-induced STAT5 activation in erythroid progenitors are recorded as measured by flow cytometry.

### Regulatory Considerations

This research is done in compliance with the applicable State and Federal laws and regulations and in compliance with ICH guidelines. The data and safety plan is executed in accordance with ICH guidelines and in compliance with policy and procedures at the H. Lee Moffitt Cancer Center and Research Institute. The following are observed to comply with Food and Drug Administration (FDA) regulations for the conduct and monitoring of clinical investigations; they also represent sound research practice:
- Informed Consent
   o The principles of informed consent are described by Federal Regulatory Guidelines (Federal Register Vol. 46, No. 17, January 27, 1981, part 50) and the Office for Protection from Research Risks Reports: Protection of Human Subjects (Code of Federal Regulations 45 CFR 46). They must be followed to comply with FDA regulations for the conduct and monitoring of clinical investigations.
- Use of Specimens For Research
   ∘ The patient is free at any time in the future to decide not to provide specimens or to withdraw his/her specimens from further scientific research. Such a decision has no impact on his/her treatment or other aspects of participation in this study.
- Institutional Review
   ∘ This study is approved by an appropriate institutional review committee as defined by Federal Regulatory Guidelines (Ref. Federal Register Vol. 46, No. 17, January 27, 1981, part 56) and the Office for Protection from Research Risks Reports: Protection of Human Subjects (Code of Federal Regulations 45 CFR 46).
- Drug Accountability
   ∘ For each drug supplied for a study, an accountability ledger containing current and accurate inventory records covering receipt, dispensing, and the return of study drug supplies is maintained. Drug supplies are kept in a secure, limited access storage area under the recommended storage conditions. During the course of the study, the following information is noted on the accountability ledger; the identification code of the subject to whom drug is dispensed, the date(s) and quantity of drug dispensed to the subject, and the date(s) and quantity of drug returned by the subject; subjects are required to return empty containers to the investigator, with the return noted on the ledger. These Accountability Forms are readily available for inspection and are open to FDA inspection at any time.
- Retention of Records
   ∘ U.S. FDA regulations (21 CFR §312.62[c]) require that records and documents pertaining to the conduct of this study and the distribution of investigational drug, including CRFs, consent forms, laboratory test results, and medication inventory records, must be retained by the Principal Investigator for 2 years after marketing application approval. If no application is filed, these records must be kept 2 years after the study is discontinued and the U.S. FDA and the applicable national and local health authorities are notified.
- Study Monitoring:
   ∘ As part of the responsibilities assumed by participating in the study, adequate case records are maintained and are available for monitoring (accurate source documents and CRFs) for the subjects treated under this protocol. In addition, all administrative documents, e.g. IRB/IEC correspondence, investigational product and supplies shipment manifests, monitoring logs, or Moffitt Cancer Center/designee correspondence are maintained. The PI is primarily responsible for monitoring of adverse events, protocol violations, and other immediate protocol issues. The study coordinator collects information of subjects enrolled at Moffitt and other institutions through the use of electronic or paper AE forms, CRF forms, End of Study forms, and Informed Consent forms.
- Internal Monitoring
   ∘ Data is captured in Oncore, Moffitt's Clinical Trials Database. The Case Report Forms are reviewed by Moffitt's Internal Monitors, periodically, throughout the conduct of the trial. The monitoring includes source data verification, utilizing research subjects' medical records.
- On-site Audits
   o The Investigator should promptly notify Moffitt Cancer Center or its authorized representative of any audits scheduled by any regulatory authorities and promptly forward copies of any audit reports received to Moffitt Cancer Center or its authorized representative.
- Data & Safety Monitoring Plan
   ∘ Identification of oversight responsibility:
   ▪ The PI has primary responsibility.
   ▪ The MCC Protocol Monitoring Committee (PMC); The PMC meets monthly and reviews accrual, patterns and frequencies of all adverse events, protocol violations and when applicable, internal audit results.
- Description of internal (PI) safety review and monitoring process:
   ∘ Responsible for identifying and reviewing adverse events biweekly:
   ▪ Principal Investigator
   ▪ Study team
- To be reviewed:
   ∘ Adverse events by grade (Gr. 3 or above using CTCAE v4.03) and attribution (expected or unexpected)
   ∘ Relationship to study drug/intervention
   ∘ Application of dose finding escalation/de-escalation **rules**
   ∘ Application of study designed stopping/decision rules
   ∘ Whether the study accrual pattern warrants continuation/action
   ∘ Protocol violations
   ∘ AEs will be reported along with all other data in the Oncore database. The PI or PI designate will report all adverse events to the Clinical Research Office (CRO). The CRO will report all SAEs to CTI, and all reportable SAEs to the IRB. AE information will be entered into the CRO database. AE information will be managed by the CRO and will be made available to the PMC or appropriate monitoring body by designated members of the PMC or the study statisticians

### Drug Preparation:

LB-100 is a water soluble enantiomeric zwitterion provided as a sterile solution for intravenous administration. As formulated in monosodium glutamate, pH 10.5, it is stable for months at -20°C and for at least 8 hours at refrigerated temperatures.

Phosphate buffered saline (PBS) was used as the vehicle for LB-100 administration in preclinical efficacy studies and sodium bicarbonate 4.2% for injection was used as the vehicle for GLP toxicity studies. Only the racemate has been studied as it has been shown that the separated enantiomers racemize rapidly in solution.

The National Institutes of Health (NIH) provides a table of Equivalent Surface Area Dosage Conversion Factors below (Table 12) which provides conversion factors that account for surface area to weight ratios between species.

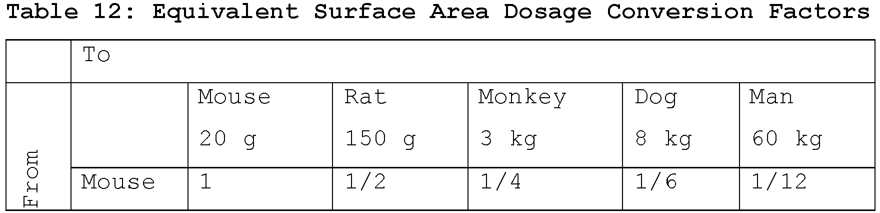

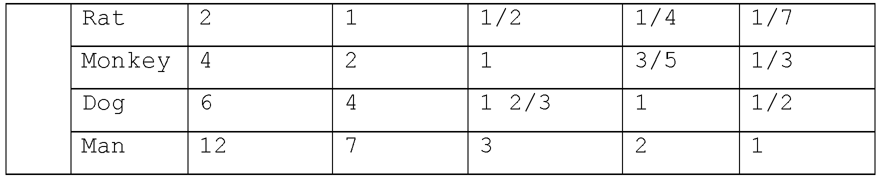

### Results

Treatment of MDS patients with del(5q) with 0.83 mg/m² of LB-100 results in hematological improvement comprising one or more of Erythroid response, Platelet response, or Neutrophil response as defined in Table 1.

Treatment of MDS patients with del(5q) with 0.83 mg/m² of LB-100 results in cytogenic response comprising either complete or partial response as defined in Table 1.

Treatment of MDS patients with del(5q) with 0.83 mg/m² of LB-100 results in complete remission as defined in Table 1.

Treatment of MDS patients with del(5q) with 0.83 mg/m² of LB-100 results in Partial remission as defined in Table 1.

Treatment of MDS patients with del(5q) with 0.83 mg/m² of LB-100 results in Marrow CR as defined in Table 1.

Treatment of MDS patients with del(5q) with 0.83 mg/m² of LB-100 results in Stable disease as defined in Table 1.

Treatment of MDS patients with del(5q) with 1.25 mg/m² of LB-100 results in hematological improvement comprising one or more of Erythroid response, Platelet response, or Neutrophil response as defined in Table 1.

Treatment of MDS patients with del(5q) with 1.25 mg/m² of LB-100 results in cytogenic response comprising either complete or partial response as defined in Table 1.

Treatment of MDS patients with del(5q) with 1.25 mg/m² of LB-100 results in complete remission as defined in Table 1.

Treatment of MDS patients with del(5q) with 1.25 mg/m² of LB-100 results in Partial remission as defined in Table 1.

Treatment of MDS patients with del(5q) with 1.25 mg/m² of LB-100 results in Marrow CR as defined in Table 1.

Treatment of MDS patients with del(5q) with 1.25 mg/m² of LB-100 results in Stable disease as defined in Table 1.

Treatment of MDS patients with del(5q) with 1.75 mg/m² of LB-100 results in hematological improvement comprising one or more of Erythroid response, Platelet response, or Neutrophil response as defined in Table 1.

Treatment of MDS patients with del(5q) with 1.75 mg/m² of LB-100 results in cytogenic response comprising either complete or partial response as defined in Table 1.

Treatment of MDS patients with del(5q) with 1.75 mg/m² of LB-100 results in complete remission as defined in Table 1.

Treatment of MDS patients with del(5q) with 1.75 mg/m² of LB-100 results in Partial remission as defined in Table 1.

Treatment of MDS patients with del(5q) with 1.75 mg/m² of LB-100 results in Marrow CR as defined in Table 1.

Treatment of MDS patients with del(5q) with 1.75 mg/m² of LB-100 results in Stable disease as defined in Table 1.

Treatment of MDS patients with del(5q) with 2.33 mg/m² of LB-100 results in hematological improvement comprising one or more of Erythroid response, Platelet response, or Neutrophil response as defined in Table 1.

Treatment of MDS patients with del(5q) with 2.33 mg/m² of LB-100 results in cytogenic response comprising either complete or partial response as defined in Table 1.

Treatment of MDS patients with del(5q) with 2.33 mg/m² of LB-100 results in complete remission as defined in Table 1.

Treatment of MDS patients with del(5q) with 2.33 mg/m² of LB-100 results in Partial remission as defined in Table 1.

Treatment of MDS patients with del(5q) with 2.33 mg/m² of LB-100 results in Marrow CR as defined in Table 1.

Treatment of MDS patients with del(5q) with 2.33 mg/m² of LB-100 results in Stable disease as defined in Table 1.

Treatment of MDS patients without del(5q) with 0.83 mg/m² of LB-100 results in hematological improvement comprising one or more of Erythroid response, Platelet response, or Neutrophil response as defined in Table 1.

Treatment of MDS patients without del(5q) with 0.83 mg/m² of LB-100 results in cytogenic response comprising either complete or partial response as defined in Table 1.

Treatment of MDS patients without del(5q) with 0.83 mg/m² of LB-100 results in complete remission as defined in Table 1.

Treatment of MDS patients without del(5q) with 0.83 mg/m² of LB-100 results in Partial remission as defined in Table 1.

Treatment of MDS patients without del(5q) with 0.83 mg/m² of LB-100 results in Marrow CR as defined in Table 1.

Treatment of MDS patients without del(5q) with 0.83 mg/m² of LB-100 results in Stable disease as defined in Table 1.

Treatment of MDS patients without del(5q) with 1.25 mg/m² of LB-100 results in hematological improvement comprising one or more of Erythroid response, Platelet response, or Neutrophil response as defined in Table 1.

Treatment of MDS patients without del(5q) with 1.25 mg/m² of LB-100 results in cytogenic response comprising either complete or partial response as defined in Table 1.

Treatment of MDS patients without del(5q) with 1.25 mg/m² of LB-100 results in complete remission as defined in Table 1.

Treatment of MDS patients without del(5q) with 1.25 mg/m² of LB-100 results in Partial remission as defined in Table 1. Treatment of MDS patients without del(5q) with 1.25 mg/m² of LB-100 results in Marrow CR as defined in Table 1.

Treatment of MDS patients without del(5q) with 1.25 mg/m² of LB-100 results in Stable disease as defined in Table 1.

Treatment of MDS patients without del(5q) with 1.75 mg/m² of LB-100 results in hematological improvement comprising one or more of Erythroid response, Platelet response, or Neutrophil response as defined in Table 1.

Treatment of MDS patients without del(5q) with 1.75 mg/m² of LB-100 results in cytogenic response comprising either complete or partial response as defined in Table 1.

Treatment of MDS patients without del(5q) with 1.75 mg/m² of LB-100 results in complete remission as defined in Table 1.

Treatment of MDS patients without del(5q) with 1.75 mg/m² of LB-100 results in Partial remission as defined in Table 1.

Treatment of MDS patients without del(5q) with 1.75 mg/m² of LB-100 results in Marrow CR as defined in Table 1.

Treatment of MDS patients without del(5q) with 1.75 mg/m² of LB-100 results in Stable disease as defined in Table 1.

Treatment of MDS patients without del(5q) with 2.33 mg/m² of LB-100 results in hematological improvement comprising one or more of Erythroid response, Platelet response, or Neutrophil response as defined in Table 1.

Treatment of MDS patients without del(5q) with 2.33 mg/m² of LB-100 results in cytogenic response comprising either complete or partial response as defined in Table 1.

Treatment of MDS patients without del(5q) with 2.33 mg/m² of LB-100 results in complete remission as defined in Table 1.

Treatment of MDS patients without del(5q) with 2.33 mg/m² of LB-100 results in Partial remission as defined in Table 1.

Treatment of MDS patients without del(5q) with 2.33 mg/m² of LB-100 results in Marrow CR as defined in Table 1.

Treatment of MDS patients without del(5q) with 2.33 mg/m² of LB-100 results in Stable disease as defined in Table 1.

### Discussion

### Myelodysplastic Syndromes

Myelodysplastic syndromes (MDS) represent both a clinical and genetically heterogeneous group of clonal hematopoietic stem cell disorders characterized by progressive cytopenias, dysplasia, and risk of transformation into acute myeloid leukemia (AML) (Greenberg, P.L. et al., 2013; Cazzola, M. et al., 2013; Greenberg, P.L. et al., 2012). MDS with isolated chromosome 5q deletion (del(5q)) represents a distinct clinical and pathological entity recognized in the World Health Organization (WHO) classification. An interstitial deletion involving chromosome 5q is the most common cytogenetic abnormality in MDS, accounting for approximately 15% of MDS cases (Haase, D. et al., 2007; Kulasekararaj, A.G. et al., 2013). Of these, half have isolated del(5q) while the remaining have either an additional cytogenetic abnormality or a complex karyotype (Haase, D. et al., 2007; Mallo, M. et al., 2011). Del(5q) MDS is characterized by hypoproliferative anemia with dysplastic megakaryocytes and a rather indolent clinical course (Giagounidis, A.A. et al., 2004).

Prognostic scoring systems have been developed to help stratify patients based on predicted survival and progression to AML (Greenberg, P.L. et al., 2012; Greenberg, P. et al., 1997; Kantarjian, H. et al., 2008). In practice, treatment decisions are made by grouping patients with MDS into lower and higher risk subgroups as defined by the International Prognostic Scoring System (IPSS, see Table 7), revised-IPSS (R-IPSS) and MD Anderson Scoring System (MDASC) (Greenberg, P.L. et al., 2012; Greenberg, P. et al., 1997; Kantarjian, H. et al., 2008). IPSS is subdivided by low, intermediate 1 (int-1), int-2, and high risk with low/int-1 risk disease representing lower risk MDS. Survival in MDS patients is generally poor, and current therapeutic options are limited (Bodor, C. et al., 2007). For those that present with lower risk disease and no deletion of 5q (del(5q)), supportive transfusions and growth factors are often utilized.

Lenalidomide (Revlimid™) and hypomethylating agents (HMA) including azacitidine (Vidaza™) and decitabine (Dacogen™) are often offered to decrease the transfusion burden of patients, but have not altered the natural history of the disease or prolonged survival (in contrast to del(5q) patients treated with lenalidomide, see below).

Furthermore, the responses are often short lived with limited alternative therapeutic options. Allogeneic stem cell transplant (ASCT) remains the only potential curative therapy. However, most subjects are ineligible secondary to age related exclusion. Those that can undergo transplant face a high degree of morbidity and unacceptable transplant related mortality with only a small fraction of subjects alive at 5 years (Lim, Z. et al., 2010). Together, patients with lower risk MDS who have progressed after initial therapy represent a group of patients with an unmet medical need.

### Lenalidomide and Del(5q) MDS

Lenalidomide represents the 1st therapeutic agent in MDS which targets a cytogenetically defined disease subset and represents the standard of care for this patient population. The initial evidence of its clinical activity was based on a high clinical and cytogenetic response rate in del(5q) MDS patients in the initial safety and efficacy study (11). Lenalidomide was approved by the Food and Drug Administration (FDA) in 2005 for the treatment of transfusion-dependent IPSS low or int-1, del(5q) MDS. The approval was based on results of the MDS-003 multicenter Phase 2 trial in which 67% of patients achieved transfusion independence (TI) with lenalidomide therapy with a median TI duration of 2.2 years (12).

In addition, 73% of patients had at least a partial cytogenetic response with 45% of patients achieving a complete response (CR). A recently published long term follow up of this study found that the median overall survival was significantly increased in patients who reached TI, 4.3 years vs. 2 years in non-responders, and in cytogenetic responders, 4.9 vs 3.1 years, respectively (13). Achievement of transfusion independence or cytogenetic response also led to a decreased risk of progression to AML.

### PP2Acα: Critical Target of Lenalidomide

Just as allelic haplodeficiency of specific genes accounts for the del(5q) MDS phenotype (Ebert, B.L. et al., 2008; Kumar, M.S. et al., 2011), gene dosage of two dual specificity phosphatases encoded within or adjacent to the proximal common deleted region (CDR) at 5q31, *CDC25C* and *PP2Acα,* underlies the selective suppression of del(5q) clones by lenalidomide (Wei, S. et al., 2009) .

Specifically, we have shown that lenalidomide induces apoptosis and cell cycle arrest in del(5q) patients but not in non-del(5q) patients. In addition to clonal suppression, lenalidomide rescues erythropoiesis through MDM2 stabilization, p53 downregulation, and release of erythroid precursors from G1 arrest (Wei, S. et al., 2013). The underlying mechanism of this effect is also through *PP2Acα* inhibition with increased phosphorylation at critical regulatory sites at serine (Ser166) and Ser186, inhibiting autoubiquitination of MDM2 thereby leading to MDM2 nuclear translocation and p53 degradation. Together, these results elucidate lenalidomide's dual ability for clonal suppression and restoration of normal erythropoiesis.

Unfortunately, approximately 50% of patients develop resistance to lenalidomide after 2-3 years of treatment and there are currently no alternative karyotype selective therapeutic agents (List, A.F. et al., 2006; List, A.F. et al., 2014) . Given that *PP2Acα* over-expression underlies lenalidomide resistance, novel strategies targeting this pathway are of pivotal importance.

One potential strategy is development of more potent and specific inhibitors of PP2A. This is reinforced by our findings that duration of TI to lenalidomide was directly related to the magnitude of PP2Acα suppression (Wei, S. et al., 2013). Specifically, median duration of TI was not reached in patients with PP2Acα suppression from baseline (1507+ days) versus 679 days in patients without (P=0.006, log rank).

In non-del(5q) MDS, lenalidomide enhances erythroid receptor signaling to restore effective erythropoiesis in a subset of patients (List, A. et al., 2005). The molecular mechanisms underlying the beneficial effects of lenalidomide in non-del(5q) cells are not clearly understood.

However, our laboratory has shown that PP2A inhibition promotes coalescence of lipid rafts with attendant incorporation and upregulation of the erythropoietin receptor along with its signaling intermediates to yield a more efficient receptor signaling platform (McGraw, K.L. et al., 2014).

Taken together, these studies characterize PP2A as an attractive therapeutic target in the treatment of lower risk MDS patients.

### Discussion of Study Results

The results of the current study indicates that the usage of LB-100 to treat human subjects suffering from MDS, whether with or without the del(5q) chromosomal abnormality, is effective.

### REFERENCES

1. Greenberg, P.L., et al. Myelodysplastic syndromes. Journal of the National Comprehensive Cancer Network : JNCCN 11, 838-874 (2013).
2. Cazzola, M., et al. The genetic basis of myelodysplasia and its clinical relevance. Blood 122, 4021-4034 (2013).
3. Greenberg, P.L., et al. Revised International Prognostic Scoring System (IPSS-R) for myelodysplastic syndromes. Blood (2012).
4. Haase, D., et al. New insights into the prognostic impact of the karyotype in MDS and correlation with subtypes: evidence from a core dataset of 2124 patients. Blood 110, 4385-4395 (2007).
5. Kulasekararaj, A.G., et al. TP53 mutations in myelodysplastic syndrome are strongly correlated with aberrations of chromosome 5, and correlate with adverse prognosis. British journal of haematology 160, 660-672 (2013).
6. Mallo, M., et al. Impact of adjunct cytogenetic abnormalities for prognostic stratification in patients with myelodysplastic syndrome and deletion 5q. Leukemia 25, 110-120 (2011).
7. Giagounidis, A.A., et al. Clinical, morphological, cytogenetic, and prognostic features of patients with myelodysplastic syndromes and del(5q) including band q31. Leukemia 18, 113-119 (2004).
8. Greenberg, P., et al. International scoring system for evaluating prognosis in myelodysplastic syndromes. Blood 89, 2079-2088 (1997).
9. Kantarjian, H., et al. Proposal for a new risk model in myelodysplastic syndrome that accounts for events not considered in the original International Prognostic Scoring System. Cancer 113, 1351-1361 (2008).
10. Bodor, C., et al. Elevated expression of Cu, Zn-SOD and Mn-SOD mRNA in inflamed dental pulp tissue. International endodontic journal 40, 128-132 (2007).
11. Lim, Z., et al. Allogeneic Hematopoietic Stem-Cell Transplantation for Patients 50 Years or Older With Myelodysplastic Syndromes or Secondary Acute Myeloid Leukemia. Journal of Clinical Oncology 28, 405-411 (2010).
12. Ebert, B.L., et al. Identification of RPS14 as a 5q- syndrome gene by RNA interference screen. Nature 451, 335-339 (2008).
13. Kumar, M.S., et al. Coordinate loss of a microRNA and protein-coding gene cooperate in the pathogenesis of 5q- syndrome. Blood 118, 4666-4673 (2011).
14. Wei, S., et al. A critical role for phosphatase haplodeficiency in the selective suppression of deletion 5q MDS by lenalidomide. Proceedings of the National Academy of Sciences of the United States of America 106, 12974-12979 (2009).
15. Wei, S., et al. Lenalidomide promotes p53 degradation by inhibiting MDM2 autoubiquitination in myelodysplastic syndrome with chromosome 5q deletion. Oncogene 32, 1110-1120 (2013).
16. List, A.F., et al. Lenalidomide (CC-5013; Revlimid(R)) Promotes Erythropoiesis in Myelodysplastic Syndromes (MDS) by CD45 Protein Tyrosine Phosphatase (PTP) Inhibition. ASH Annual Meeting Abstracts 108, 1360- (2006).
17. List, A.F., et al. Extended survival and reduced risk of AML progression in erythroid-responsive lenalidomide-treated patients with lower-risk del(5q) MDS. Leukemia 28, 1033-1040 (2014).
18. List, A., et al. Efficacy of lenalidomide in myelodysplastic syndromes. The New England journal of medicine 352, 549-557 (2005) .
19. McGraw, K.L., et al. Lenalidomide induces lipid raft assembly to enhance erythropoietin receptor signaling in myelodysplastic syndrome progenitors. PloS one 9, e114249 (2014).
20. Hart, M.E., et al. Modified norcantharidins; synthesis, protein phosphatases 1 and 2A inhibition, and anticancer activity. Bioorganic & medicinal chemistry letters 14, 1969-1973 (2004).
21. Lu, J., et al. Inhibition of serine/threonine phosphatase PP2A enhances cancer chemotherapy by blocking DNA damage induced defense mechanisms. Proceedings of the National Academy of Sciences of the United States of America 106, 11697-11702 (2009).
22. Zhuang, Z., et al. Enhancement of cancer chemotherapy by simultaneously altering cell cycle progression and DNAdamage defenses through global modification of the serine/threonine phosphoproteome. Cell cycle 8, 3303-3306 (2009).
23. Zhang, C., et al. A synthetic cantharidin analog for the enhancement of doxorubicin suppression of stem cell-derived aggressive sarcoma. Biomaterials 31, 9535-9543 (2010).
24. Lu, J., et al. The effect of a PP2A inhibitor on the nuclear receptor corepressor pathway in glioma. Journal of neurosurgery 113, 225-233 (2010).
25. Vincent M. Chung, A.S.M., John Kovach. A phase 1 study of a novel inhibitor of protein phosphatase 2A alone and with docetaxel. Journal of clinical oncology : official journal of the American Society of Clinical Oncology 32, suppl; abstr TPS2636 (2014).
26. Cheson, B.D., et al. Clinical application and proposal for modification of the International Working Group (IWG) response criteria in myelodysplasia. Blood 108, 419-425 (2006).
27. Vardiman, J.W., et al. The 2008 revision of the World Health Organization (WHO) classification of myeloid neoplasms and acute leukemia: rationale and important changes. Blood 114, 937-951 (2009).

## Claims

1. A compound having the structure 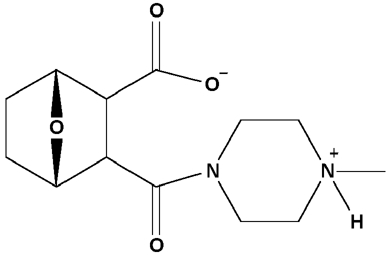 or a salt, zwitterion, or ester thereof for use in treating myelodysplastic syndrome in a human subject afflicted therewith, said use comprising administering to the subject an amount from 0.1 mg/m² to 5 mg/m² of said compound, wherein
(i) the human subject is afflicted with myelodysplastic syndrome with isolated chromosome 5q deletion or without isolated chromosome 5q deletion and has previously undergone failed prior treatment with at least 2 cycles of lenalidomide, or
(ii) the human subject has further previously undergone failed prior treatment with at least 2 cycles of azacitidine and/or decitabine.

2. The compound for use of claim 1, wherein the amount of the compound administered is about 0.25 mg/m², 0.5 mg/m², 0.75 mg/m², 1.0 mg/m², 1.25 mg/m², 1.5 mg/m², 1.75 mg/m², 2.0 mg/m², 2.25 mg/m², 2.5 mg/m² or 2.75 mg/m²; or:
wherein the amount of the compound administered is 0.25 mg/m² to 2.5 mg/m²; optionally:
wherein the amount of the compound administered is 0.25 mg/m², 0.5 mg/m², 0.83 mg/m², 1.25 mg/m², 1.75 mg/m² or 2.33 mg/m²; or:
wherein the amount of the compound administered is 2.5 mg/m² to 5 mg/m²; or:
wherein the amount of the compound administered is 3 mg/m² to 4.5 mg/m²; optionally:
wherein the amount of the compound administered is about 3 mg/m², 3.25 mg/m², 3.5 mg/m², 3.75 mg/m², 4 mg/m², 4.25 mg/m² or 4.5 mg/m²; or:
wherein the amount of the compound administered is 0.83 mg/m² to 2.33 mg/m²; optionally:
wherein the amount of the compound administered is 0.83 mg/m², 1.25 mg/m², 1.75 mg/m², or 2.33 mg/m².

3. The compound for use of claim 1 or claim 2, wherein the amount of the compound is administered once daily; or:
wherein the amount of the compound is administered once daily for a three day period; or:
wherein the amount of the compound is administered three times per week; or:
wherein the amount of the compound is administered on three separate days during a seven day period; or:
wherein the amount of the compound is administered on three separate days during a twenty-one day treatment cycle; or:
wherein the amount of the compound is administered on three separate days during week 1 of a twenty-one day treatment cycle; optionally:
wherein the amount of the compound is administered on days 1, 2 and 3 of a twenty-one day treatment cycle; or:
wherein the amount of the compound is administered on days 1, 2 and 3 of a twenty-one day treatment cycle and the cycle is repeated one or more times; or:
wherein the amount of the compound is administered on days 1, 2 and 3 of a twenty-one day treatment cycle and the cycle is repeated one or more times; or:
wherein the amount of the compound is administered on days 1, 2 and 3 of a twenty-one day treatment cycle and the cycle is repeated two or more times; or:
wherein the amount of the compound is administered on days 1, 2 and 3 of a twenty-one day treatment cycle and the cycle is repeated three or more times; or:
wherein the amount of the compound is administered on days 1, 2 and 3 of a twenty-one day treatment cycle and the cycle is repeated four or more times; or:
wherein the amount of the compound is administered on days 1, 2 and 3 of a twenty-one day treatment cycle and the cycle is repeated five or more times; or:
wherein the amount of the compound is administered on days 1, 2 and 3 of a twenty-one day treatment cycle and the cycle is repeated six or more times; or:
wherein the amount of the compound is administered on days 1, 2 and 3 of a twenty-one day treatment cycle and the cycle is repeated between 1 to 10 times.

4. The compound for use of any of claims 1 to 3, wherein the use in treating comprises achievement of hematological improvement in the human subject.

5. The compound for use of claim 4, wherein the hematological improvement comprises an erythroid response, wherein the erythroid response comprises an Hgb increase by ≥ 1.5 g/dL, and there is a relevant reduction of units of RBC transfusions by an absolute number of at least 4 RBC transfusions/8 weeks compared with the pretreatment transfusion number in the previous 8 weeks, wherein only RBC transfusions given for a Hgb of ≤ 9.0 g/dL pretreatment will count in the RBC transfusion evaluation; or:
wherein the hematological improvement comprises a platelet response, wherein the platelet response comprises an absolute increase of ≥ 30 x 10⁹/L platelets for subjects starting with > 20 x 10⁹/L platelets, or an increase from < 20 x 10⁹/L platelets to > 20 x 10⁹/L platelets, wherein the increase is by a proportion of at least 100%; or:
wherein the hematological improvement comprises a neutrophil response, wherein
the neutrophil response comprises at least a 100% increase in neutrophils, wherein
the increase is an absolute increase of > 0.5 x 10⁹/L.

6. The compound for use of any one of claims 1 to 3, wherein the use in treating comprises achievement of a cytogenic response in the human subject.

7. The compound for use of claim 6, wherein the cytogenic response comprises a complete response, wherein the complete response comprises a disappearance of the chromosomal abnormality without appearance of new abnormalities; or:
wherein the cytogenic response comprises a partial response, wherein the partial response comprises at least a 50% reduction of the chromosomal abnormality.

8. The compound for use of any of claims 1 to 3, wherein the use in treating comprises a complete remission of MDS in the human subject; optionally:
wherein the complete remission comprises achievement of ≤ 5% myeloblasts in the bone marrow with normal maturation of all cell lines, and achievement of hemoglobin ≥ 11 g/dL, platelets ≥ 100 x 10⁹/L, neutrophils ≥ 1.0 x 10⁹/L, and 0% blasts in peripheral blood; or:
wherein the use in treating comprises a partial remission of MDS in the human subject; optionally:
wherein the partial remission comprises achievement of a decrease of myeloblasts in the bone marrow of ≥ 50% over pretreatment, with normal maturation of all cell lines, wherein the level of myeloblasts in the bone marrow is > 5%, and achievement of hemoglobin ≥ 11 g/dL, platelets ≥ 100 x 10⁹/L, neutrophils ≥ 1.0 x 10⁹/L, and 0% blasts in peripheral blood; or:
wherein the use in treating comprises marrow complete remission of MDS in the human subject; optionally:
wherein the marrow complete remission comprises achievement of a decrease of myeloblasts in the bone marrow of ≥ 50% over pretreatment, wherein the level of myeloblasts in the bone marrow is ≤ 5%; or:
wherein the use in treating comprises stabilization of MDS in the human subject; optionally:
wherein the stabilization of MDS comprises failure to achieve a decrease of myeloblasts of ≥ 50% over pretreatment, failure to achieve ≤ 5% myeloblasts, or failure to achieve normal maturation of all cell lines, in the bone marrow, or failure to achieve hemoglobin ≥ 11 g/dL, platelets ≥ 100 x 10⁹/L, neutrophils ≥ 1.0 x 10⁹/L, or 0% blasts in peripheral blood, and wherein the human subject exhibits no evidence of progression of the disease for > 8 weeks.

## Patentansprüche

1. Verbindung mit der Struktur: 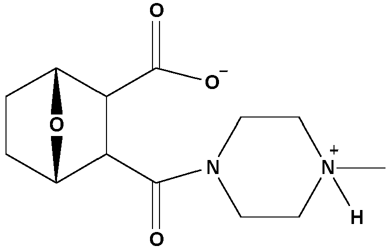 oder ein Salz, Zwitterion oder Ester davon zur Verwendung bei der Behandlung des myelodysplastischen Syndroms in einem daran leidenden Menschen, wobei die Verwendung die Verabreichung einer Menge von 0,1 mg/m² bis 5 mg/m² der Verbindung an den Menschen umfasst, wobei
(i) der Mensch an einem myelodysplastischen Syndrom mit isolierter Deletion des Chromosoms 5q oder ohne isolierte Deletion des Chromosoms 5q leidet und zuvor einer fehlgeschlagen vorherigen Behandlung mit mindestens 2 Zyklen Lenalidomid unterzogen wurde, oder
(ii) der Mensch zuvor einer fehlgeschlagenen vorherigen Behandlung mit mindestens 2 Zyklen Azacitidin und/oder Decitabin unterzogen wurde.

2. Verbindung zur Verwendung nach Anspruch 1, wobei die verabreichte Menge der Verbindung etwa 0,25 mg/m², 0,5 mg/m², 0,75 mg/m², 1,0 mg/m², 1,25 mg/m², 1,5 mg/m², 1,75 mg/m², 2,0 mg/m², 2,25 mg/m², 2,5 mg/m² oder 2,75 mg/m² beträgt; oder:
wobei die verabreichte Menge der Verbindung 0,25 mg/m² bis 2,5 mg/m² beträgt; gegebenenfalls:
wobei die verabreichte Menge der Verbindung 0,25 mg/m², 0,5 mg/m², 0,83 mg/m², 1,25 mg/m², 1,75 mg/m² oder 2,33 mg/m² beträgt; oder:
wobei die verabreichte Menge der Verbindung 2,5 mg/m² bis 5 mg/m² beträgt; oder:
wobei die verabreichte Menge der Verbindung 3 mg/m² bis 4,5 mg/m² beträgt; gegebenenfalls:
wobei die verabreichte Menge der Verbindung etwa 3 mg/m², 3,25 mg/m², 3,5 mg/m², 3,75 mg/m², 4 mg/m², 4,25 mg/m² oder 4.5 mg/m² beträgt; oder:
wobei die verabreichte Menge der Verbindung 0,83 mg/m² bis 2,33 mg/m² beträgt; gegebenenfalls:
wobei die verabreichte Menge der Verbindung 0,83 mg/m², 1,25 mg/m², 1,75 mg/m² oder 2,33 mg/m².

3. Verbindung zur Verwendung nach Anspruch 1 oder Anspruch 2, wobei die Menge der Verbindung einmal täglich verabreicht wird; oder:
wobei die Menge der Verbindung einmal täglich über einen Zeitraum von drei Tagen verabreicht wird; oder:
wobei die Menge der Verbindung dreimal pro Woche verabreicht wird; oder:
wobei die Menge der Verbindung an drei separaten Tagen während eines Zeitraums von sieben Tagen verabreicht wird; oder:
wobei die Menge der Verbindung an drei separaten Tagen während eines 21-tägigen Behandlungszyklus verabreicht wird; oder:
wobei die Menge der Verbindung an drei separaten Tagen während der Woche 1 eines 21-tägigen Behandlungszyklus verabreicht wird; gegebenenfalls:
wobei die Menge der Verbindung an den Tagen 1, 2 und 3 eines 21-tägigen Behandlungszyklus verabreicht wird; oder:
wobei die Menge der Verbindung an den Tagen 1, 2 und 3 eines 21-tägigen Behandlungszyklus verabreicht wird und der Zyklus einmal oder öfter wiederholt wird; oder:
wobei die Menge der Verbindung an den Tagen 1, 2 und 3 eines 21-tägigen Behandlungszyklus verabreicht wird und der Zyklus einmal oder öfter wiederholt wird; oder:
wobei die Menge der Verbindung an den Tagen 1, 2 und 3 eines 21-tägigen Behandlungszyklus verabreicht wird und der Zyklus zweimal oder öfter wiederholt wird; oder:
wobei die Menge der Verbindung an den Tagen 1, 2 und 3 eines 21-tägigen Behandlungszyklus verabreicht wird und der Zyklus dreimal oder öfter wiederholt wird; oder:
wobei die Menge der Verbindung an den Tagen 1, 2 und 3 eines 21-tägigen Behandlungszyklus verabreicht wird und der Zyklus viermal oder öfter wiederholt wird; oder:
wobei die Menge der Verbindung an den Tagen 1, 2 und 3 eines 21-tägigen Behandlungszyklus verabreicht wird und der Zyklus fünfmal oder öfter wiederholt wird; oder:
wobei die Menge der Verbindung an den Tagen 1, 2 und 3 eines 21-tägigen Behandlungszyklus verabreicht wird und der Zyklus sechsmal oder öfter wiederholt wird; oder:
wobei die Menge der Verbindung an den Tagen 1, 2 und 3 eines 21-tägigen Behandlungszyklus verabreicht wird und der Zyklus ein- bis zehnmal wiederholt wird.

4. Verbindung zur Verwendung nach einem der Ansprüche 1 bis 3, wobei die Verwendung bei der Behandlung das Erreichen einer hämatologischen Verbesserung beim Menschen umfasst.

5. Verbindung zur Verwendung nach Anspruch 4, wobei die hämatologische Verbesserung eine erythroide Reaktion umfasst, wobei die erythroide Reaktion einen Hgb-Anstieg von ≥ 1,5 g/dL umfasst und eine relevante Verringerung der Einheiten von RBC-Transfusionen um eine absolute Zahl von mindestens 4 RBC-Transfusionen/8 Wochen im Vergleich zur Transfusionszahl vor der Behandlung in den letzten 8 Wochen vorliegt, wobei nur RBC-Transfusionen, die bei einem Hgb von ≤ 9,0 g/dL vor der Behandlung verabreicht wurden, in die RBC-Transfusionsbewertung eingehen; oder:
wobei die hämatologische Verbesserung eine Thrombozytenantwort umfasst,
wobei die Thrombozytenantwort einen absoluten Anstieg von ≥ 30 x 10⁹/L Thrombozyten für Patienten umfasst, die mit > 20 x 10⁹/L Thrombozyten beginnen,
oder einen Anstieg von < 20 x 10⁹/L Thrombozyten auf > 20 x 10⁹/L Thrombozyten umfasst, wobei die Zunahme um einen Anteil von mindestens 100% erfolgt; oder:
wobei die hämatologische Verbesserung eine Neutrophilen-Antwort umfasst, wobei die Neutrophilen-Antwort eine mindestens 100%ige Zunahme an Neutrophilen umfasst, wobei die Zunahme eine absolute Zunahme von > 0,5 x 10⁹/L ist.

6. Verbindung zur Verwendung nach einem der Ansprüche 1 bis 3, wobei die Verwendung bei der Behandlung das Erreichen einer zytogenen Antwort beim Menschen umfasst.

7. Verbindung zur Verwendung nach Anspruch 6, wobei die zytogene Antwort eine vollständige Antwort umfasst, wobei die vollständige Antwort ein Verschwinden der chromosomalen Anomalie ohne Auftreten neuer Anomalien umfasst; oder:
wobei die zytogene Antwort eine Teilantwort umfasst, wobei die Teilantwort eine mindestens 50%ige Reduktion der chromosomalen Anomalie umfasst.

8. Verbindung zur Verwendung nach einem der Ansprüche 1 bis 3, wobei die Verwendung bei der Behandlung eine vollständige Remission von MDS beim Menschen umfasst; gegebenenfalls:
wobei die vollständige Remission das Erreichen von ≤ 5% Myeloblasten im Knochenmark bei normaler Reifung aller Zelllinien und das Erreichen von Hämoglobin ≥ 11 g/dL, Thrombozyten ≥ 100 x 10⁹/L, Neutrophilen ≥ 1,0 x 10⁹/L und 0% Blasten im peripheren Blut umfasst; oder:
wobei die Verwendung bei der Behandlung eine teilweise Remission von MDS beim Menschen umfasst; gegebenenfalls:
wobei die teilweise Remission das Erreichen einer Abnahme der Myeloblasten im Knochenmark von ≥ 50 % gegenüber der Vorbehandlung mit normaler Reifung aller Zelllinien umfasst, wobei der Myeloblastenspiegel im Knochenmark > 5% beträgt und Hämoglobin ≥ 11 g/dL, Thrombozyten ≥ 100 x 10⁹/L, Neutrophile ≥ 1,0 x 10⁹/L und 0% Blasten im peripheren Blut erreicht wird; oder:
wobei die Verwendung bei der Behandlung eine vollständige Remission von MDS im Knochenmark beim Menschen umfasst; gegebenenfalls:
wobei die vollständige Remission im Knochenmark das Erreichen einer Abnahme von Myeloblasten im Knochenmark von ≥ 50% gegenüber der Vorbehandlung umfasst, wobei der Myeloblastenspiegel im Knochenmark ≤ 5% beträgt; oder:
wobei die Verwendung bei der Behandlung die Stabilisierung von MDS beim Menschen umfasst; gegebenenfalls:
wobei die Stabilisierung von MDS darin besteht, dass im Knochenmark die Myeloblasten im Vergleich zur Vorbehandlung nicht um ≥ 50 % gesenkt werden, Myeloblasten von ≤ 5% nicht erreicht werden, oder die normale Reifung aller Zelllinien nicht erreicht wird, oder im peripheren Blut Hämoglobin von ≥ 11 g/dL, Thrombozyten von ≥ 100 x 10⁹/L, Neutrophile von ≥ 1,0 x 10⁹/L oder 0% Blasten nicht erreicht werden, und wobei der Mensch für > 8 Wochen keinen Hinweis auf ein Fortschreiten der Krankheit zeigt.

## Revendications

1. Composé ayant la structure 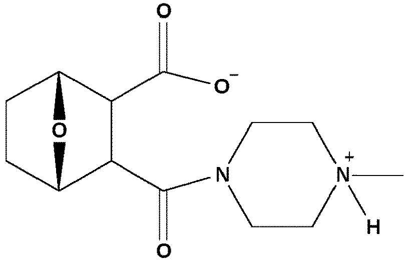 ou un sel, un zwitterion ou un ester de celui-ci pour son utilisation dans le traitement du syndrome myélodysplasique chez un sujet humain qui en est atteint, ladite utilisation comprenant l'administration au sujet d'une quantité de 0,1 mg/m² à 5 mg/m² dudit composé, dans lequel
(i) le sujet humain est atteint d'un syndrome myélodysplasique avec délétion isolée du chromosome 5q ou sans délétion isolée du chromosome 5q et a précédemment subi un traitement antérieur ayant échoué avec au moins 2 cycles de lénalidomide, ou
(ii) le sujet humain a en outre subi un traitement antérieur ayant échoué avec au moins 2 cycles d'azacitidine et/ou de décitabine.

2. Composé pour son utilisation selon la revendication 1,
dans lequel la quantité du composé administré est d'environ 0,25 mg/m², 0,5 mg/m², 0,75 mg/m², 1,0 mg/m², 1,25 mg/m², 1,5 mg/m², 1,75 mg/m², 2,0 mg/m², 2,25 mg/m², 2,5 mg/m² ou 2,75 mg/m² ; ou :
dans lequel la quantité du composé administré est de 0,25 mg/m² à 2,5 mg/m² ; éventuellement :
dans lequel la quantité du composé administré est de 0,25 mg/m², 0,5 mg/m², 0,83 mg/m², 1,25 mg/m², 1,75 mg/m² ou 2,33 mg/m² ; ou :
dans lequel la quantité du composé administré est de 2,5 mg/m² à 5 mg/m² ; ou :
dans lequel la quantité du composé administré est de 3 mg/m² à 4,5 mg/m² ; éventuellement :
dans lequel la quantité du composé administré est d'environ 3 mg/m², 3,25 mg/m², 3,5 mg/m², 3,75 mg/m², 4 mg/m², 4,25 mg/m² ou 4,5 mg/m² ; ou :
dans lequel la quantité du composé administré est de 0,83 mg/m² à 2,33 mg/m² ; éventuellement :
dans lequel la quantité du composé administré est de 0,83 mg/m², 1,25 mg/m², 1,75 mg/m² ou 2,33 mg/m².

3. Composé pour son utilisation selon la revendication 1 ou la revendication 2, dans lequel la quantité du composé est administrée une fois par jour; ou :
dans lequel la quantité du composé est administrée une fois par jour pendant une période de trois jours ; ou :
dans lequel la quantité du composé est administrée trois fois par semaine ; ou :
dans lequel la quantité du composé est administrée sur trois jours séparés pendant une période de sept jours ; ou :
dans lequel la quantité du composé est administrée sur trois jours séparés pendant un cycle de traitement de vingt-et-un jours ; ou :
dans lequel la quantité du composé est administrée sur trois jours séparés pendant la semaine 1 d'un cycle de traitement de vingt-et-un jours ; éventuellement :
dans lequel la quantité du composé est administrée les jours 1, 2 et 3 d'un cycle de traitement de vingt-et-un jours ; ou :
dans lequel la quantité du composé est administrée les jours 1, 2 et 3 d'un cycle de traitement de vingt-et-un jours et le cycle est répété une ou plusieurs fois ; ou :
dans lequel la quantité du composé est administrée les jours 1, 2 et 3 d'un cycle de traitement de vingt-et-un jours et le cycle est répété une ou plusieurs fois ; ou :
dans lequel la quantité du composé est administrée les jours 1, 2 et 3 d'un cycle de traitement de vingt-et-un jours et le cycle est répété deux fois ou plus ; ou :
dans lequel la quantité du composé est administrée les jours 1, 2 et 3 d'un cycle de traitement de vingt-et-un jours et le cycle est répété trois fois ou plus ; ou :
dans lequel la quantité du composé est administrée les jours 1, 2 et 3 d'un cycle de traitement de vingt-et-un jours et le cycle est répété quatre fois ou plus ; ou :
dans lequel la quantité du composé est administrée les jours 1, 2 et 3 d'un cycle de traitement de vingt-et-un jours et le cycle est répété cinq fois ou plus ; ou :
dans lequel la quantité du composé est administrée les jours 1, 2 et 3 d'un cycle de traitement de vingt-et-un jours et le cycle est répété six fois ou plus ; ou :
dans lequel la quantité du composé est administrée les jours 1, 2 et 3 d'un cycle de traitement de vingt-et-un jours et le cycle est répété entre 1 et 10 fois.

4. Composé pour son utilisation selon l'une quelconque des revendications 1 à 3, dans lequel l'utilisation dans le traitement comprend l'obtention d'une amélioration hématologique chez le sujet humain.

5. Composé pour son utilisation selon la revendication 4, dans lequel l'amélioration hématologique comprend une réponse érythroïde, dans lequel la réponse érythroïde comprend une augmentation du taux d'Hgb ≥ 1,5 g/dL, et il existe une réduction significative des unités de transfusions de globules rouges RBC par un nombre absolu d'au moins 4 transfusions de globules rouges RBC/8 semaines par rapport au nombre de transfusions dans les 8 semaines précédant le traitement, dans lequel seules les transfusions de globules rouges RBC données pour un taux prétraitement d'Hgb ≤ 9,0 g/dL sont prises en compte dans l'évaluation des transfusions de globules rouges RBC ; ou :
dans lequel l'amélioration hématologique comprend une réponse plaquettaire, dans lequel la réponse plaquettaire comprend une augmentation absolue du taux de plaquettes ≥ 30 x 10⁹/L pour des sujets présentant initialement un taux de plaquettes > 20 x 10⁹/L ou une augmentation du taux de plaquettes de < 20 x 10⁹/L à > 20 x 10⁹/L, dans lequel l'augmentation est d'au moins 100 % ; ou :
dans lequel l'amélioration hématologique comprend une réponse neutrophile, dans lequel la réponse neutrophile comprend au moins une augmentation de 100 % des neutrophiles, dans lequel l'augmentation est une augmentation absolue > 0,5 x 10⁹/L.

6. Composé pour son utilisation selon l'une quelconque des revendications 1 à 3, dans lequel l'utilisation dans le traitement comprend l'obtention d'une réponse cytogénique chez le sujet humain.

7. Composé pour son utilisation selon la revendication 6, dans lequel la réponse cytogénique comprend une réponse complète, dans lequel la réponse complète comprend une disparition de l'anomalie chromosomique sans apparition de nouvelles anomalies ; ou :
dans lequel la réponse cytogénique comprenant une réponse partielle, dans lequel la réponse partielle comprend au moins une réduction de 50 % de l'anomalie chromosomique.

8. Composé pour son utilisation selon l'une quelconque des revendications 1 à 3, dans lequel l'utilisation dans le traitement comprend une rémission complète du syndrome myélodysplasique MDS chez le sujet humain ; éventuellement :
dans lequel la rémission complète comprend l'obtention d'un taux ≤ 5 % de myéloblastes dans la moelle osseuse avec une maturation normale de toutes les lignées cellulaires, et l'obtention d'un taux d'hémoglobine ≥ 11 g/dL, de plaquettes ≥ 100 x 10⁹/L, de neutrophiles ≥ 1,0 x 10⁹/L et de 0 % de blastes dans le sang périphérique ; ou :
dans lequel l'utilisation dans le traitement comprend une rémission partielle du syndrome myélodysplasique MDS chez le sujet humain ; éventuellement :
dans lequel la rémission partielle comprend l'obtention d'une diminution des myéloblastes dans la moelle osseuse ≥ 50 % par rapport à l'état prétraitement, avec une maturation normale de toutes les lignées cellulaires, dans lequel le taux de myéloblastes dans la moelle osseuse est > 5 %, et l'obtention d'un taux d'hémoglobine ≥ 11 g/dL, de plaquettes ≥ 100 x 10⁹/L, de neutrophiles ≥ 1,0 x 10⁹/L, et de 0 % de blastes dans le sang périphérique ; ou :
dans lequel l'utilisation dans le traitement comprend la rémission complète médullaire du syndrome myélodysplasique MDS chez le sujet humain ; éventuellement :
dans lequel la rémission complète médullaire comprend l'obtention d'une diminution des myéloblastes dans la moelle osseuse ≥ 50 % par rapport à l'état prétraitement, dans lequel le taux de myéloblastes dans la moelle osseuse est ≤ 5 % ; ou :
dans lequel l'utilisation dans le traitement comprenant la stabilisation du syndrome myélodysplasique MDS chez le sujet humain ; éventuellement :
dans lequel la stabilisation du syndrome myélodysplasique MDS comprend l'échec à obtenir une diminution des myéloblastes ≥ 50 % par rapport à l'état prétraitement, l'échec à obtenir un taux ≤ 5 % de myéloblastes, ou l'échec à obtenir une maturation normale de toutes les lignées cellulaires, dans la moelle osseuse, ou l'échec à obtenir un taux d'hémoglobine ≥ 11 g/dL, de plaquettes ≥ 100 x 10⁹/L, de neutrophiles ≥ 1,0 x 10⁹/L, ou de 0 % de blastes dans le sang périphérique, et dans lequel le sujet humain ne présente pas de signe de progression de la maladie pendant plus de 8 semaines.
